# EUROPEAN PATENT APPLICATION

(11) **EP 1 997 472 A1**
(43) Date of publication of application: **03.12.2008**
(21) Application number: 08251882.0
(22) Date of filing: 30.05.2008
(51) Int. Cl.: A61K 8/19, A61K 8/25, A61K 8/26, A61K 8/27, A61K 8/29, A61K 8/34, A61K 8/41, A61K 8/86, A61Q 5/06, A61K 8/02

(54) **Cosmetic hair compositions containing metal-oxide layered pigments and functionalized metal-oxide layered pigments and methods of use**

(30) Priority: 30.05.2007 US 940792 P; 30.05.2007 US 755169
(71) Applicant: L'Oreal, 75008 Paris (FR)
(72) Inventor: Barrios, Jaimie J, Clark, NJ 07066 (US); Burakov, Dina, Edison, NJ 08837 (US); Castillo-Bucci, Carmen, Englewood, NJ 07631 (US); Henao-Cano, Uriel, Edison, NJ 08817 (US); Quadir, Murat, Scotch Plains, NJ 07076 (US)
(74) Representative: Rutherford, Jodie

(57) **Abstract**

The disclosure relates to cosmetic hair compositions containing metal-oxide layered pigments. The disclosure also relates to the use of the metal-oxide layered pigments in cosmetic hair compositions. The disclosure also relates to compositions containing film-formers and stabilizers in combination with the metal-oxide layered pigments. The disclosure also relates to cosmetic compositions containing functionalized metal-oxides layered pigments. The disclosure also relates to the use of the functionalized metal-oxide layered pigments in cosmetics such as hair, eyes, lips, skin and nail compositions. The metal-oxide layered pigments are functionalized with ligands which provide improved properties to the pigments in the cosmetics.

## Description

### TECHNICAL FIELD

The disclosure relates to cosmetic hair compositions containing metal-oxides layered pigments. The disclosure also relates to the use of the metal-oxide layered pigments in cosmetic hair compositions. The disclosure also relates to cosmetic compositions containing functionalized metal-oxides layered pigments. The disclosure also relates to the use of the functionalized metal-oxide layered pigments in cosmetics such as hair, eyes, lips, skin and nail compositions. The metal-oxide layered pigments are functionalized with ligands which provide improved properties to the pigments in the cosmetics.

### BACKGROUND OF THE DISCLOSURE

Pigments are used in cosmetic compositions to alter the color and appearance of hair, eyes, lips, skin and nails. Metal-oxide layered pigments have been used in some of these compositions because they can provide for a variety of colors. Because of poor compatibility of the metal-oxide layered pigment with other components of the cosmetic compositions, the pigments are sometimes hydrophobically modified to improve the compatibility of the pigment with oil containing cosmetic compositions. However, the unwanted transfer of these pigments in applications such as hair cosmetics is a problem. Ideally, the pigment should stay where the pigment initially applied to the hair and not be transferred to other locations such as hands, skin, clothes and furniture. In addition, while the pigment should stay on the hair, the hair composition should provide other desirable hair properties such as manageability and feel. Specifically, hair stiffness should not be adversely affected by the hair composition. In addition, while a variety of colors is available, a greater variety of colors is desirable.

### SUMMARY OF THE DISCLOSURE

The disclosure involves compositions for use in hair cosmetics. The compositions contain a metal-oxide layered pigment, optionally, at least one film-former, at least one stabilizer and at least one auxiliary ingredient where the metal-oxide layered pigment contains at least two layers. The compositions are useful in cosmetic hair compositions. The metal-oxide layered pigments provide improved optical effects when applied to hair. For example, hair color shifts depending upon viewing angle (color travel effect) and the pigments improve the shine of hair.

The disclosure also involves compositions for use in cosmetics. The compositions contain the composition described above and further comprising at least one polymer where the surface of the metal-oxide layer pigment is functionalized with a ligand by formation of at least one bond between a metal at the surface of the metal-oxide layered pigment and the ligand. The compositions are useful in cosmetics such as skin, hair, eye, lip and nail compositions. The functionalized metal-oxide layered pigment of the disclosure gives improved properties to the cosmetic formulation including improved color variety and pigment transfer properties.

The functionalized pigments give improved surface properties to the pigments such that the pigment transfer characteristics of the pigment are improved. In addition, careful selection of the ligand that functionalizes the pigment can be used to fine tune the color of the pigment. This fine tuning of color is achieved by selecting the ligand such that the ligand to metal charge transfer that occurs between the ligated metal in the metal-oxide pigment and the ligand gives the desired color. (T. Rajh et al., "Surface Restructuring of Nanoparticles: An Efficient Route for Ligand-Metal Oxide Crosstalk", J. Phys. Chem., B2002, 106, 10543-10552). This technique provides for a greater variety of colors for the metal-oxide pigments.

### DETAILED DESCRIPTION OF THE DISCLOSURE

One embodiment of the disclosure involves a composition comprising (a) a metal-oxide layered pigment, (b) optionally, at least one film-former, (c) at least one stabilizer and (d) at least one auxiliary ingredient where the metal-oxide layered pigment comprises at least two layers.

Another embodiment of the disclosure involves a composition as described above further comprising (e) at least one polymer where the surface of the metal-oxide layered pigment is functionalized with a ligand by formation of at least one bond between a metal at the surface of the metal-oxide layered pigment and the ligand.

The metal-oxide layered pigment is not limited and can be any layered metal-oxide composition containing at least two layers. Typically, the layered pigment contains from 2 to 10 layers and more typically contains 3 to 7 layers and even more typically contains 3 to 5 layers. In addition, the individual layers of the metal-oxide layered pigment have a thickness of from about 0.02 to about 5 microns. Typically, the thickness of the individual layers is from about 0.05 to about 4 microns and more typically from about 0.05 to about 0.5 microns. The particle size of the metal-oxide layered pigment depends upon the application and is typically from about 5 to 500 microns, more typically from about 10 to 100 microns and even more typically from about 30 to about 70 microns.

The metal-oxide of the metal-oxide is not limited and includes TiO₂, Fe₂O₃, CaCO₃, SnO, SnO₂, MgSiO₃, Cr₂O₃, ZnO, MgO, ZnS, ZrO₂, CuO, MgF₂, Ce₂O₃, CeO₂, Y₂O₃, CaF₂, Al₂O₃, BaSO₄, BiOCl, SiO₂, glass flake mica, talc and kaolin and optionally comprising a polymer particle. Preferably the metal oxide can be silicon dioxide and/or titanium dioxide and/or iron oxides e.g. Fe₂O₃. For example, pigments which have silicon dioxide (silica) and iron oxides or silicon dioxide (silica) and titanium dioxide are contemplated. The concentration of the metal-oxide layered pigment ranges from about 0.01 to about 3 weight % based on the total weight of the cosmetic composition. More typically, the concentration of the metal-oxide layered pigment ranges from about 0.1 to about 2 weight % based on the total weight of the cosmetic composition.

Non-limiting examples of the metal-oxide layered pigment includes pigments marketed under the trade designations Xirona^{®}, Colorona^{®}, Timiron^{®}, Dichrona^{®}, Microna^{®}, Soloron^{®}, Prestige^{®}, Flonac^{®}, Flamenco^{®}, Timica^{®}, Duochrome^{®} and mixtures thereof. Preferably, the metal-oxide layered pigment is Xirona^{®}, for example Xirona^{®} Indian Summer or Xirona^{®} Nordic Sunset.

The ligand that functionalizes the metal-oxide layered pigment is not limited and can be any compound that is capable of forming at least one bond with a metal at the surface of the metal-oxide layered pigment. Non-limiting types of compounds include catechol, a substituted catechol, electron-donating bidentate ligands, dihydroxy cyclobutenedione, ascorbic acid, methyl catechol, tertbutyl catechol, salicylic acid, a substituted salicylic acid, gallol, a substituted gallol, benzene-1,2,3-triol, a substituted benzene-1,2,3-triol, benzene-1,2,4-triol, a substituted benzene-1,2,4-triol, benzene-1,2,3,4-tetraol, a substituted benzene-1,2,3,4-tetraol, dopamine, alizarin, an organoamine, an organopolycarboxylate, an organoamino carboxylate, an organosilane, an alkoxysilane, an organophosphate, an organophosphonate, an aminophosphonate, a disulfide, a mercaptocarboxylate, an alkylthiocarbamate, an alkylcarbamate, a polyalkylene glycol/amino acid copolymer, a mPEG-DOPA copolymer, poly[(3,4-dihydroxystyrene)/styrene copolymer, a polyalkylene glycol functionalized acrylic acid, a polyalkylene glycol functionalized methacrylic acid, a gallol-PEG polymer, a compound or polymer with adjacent hydroxyl groups and mixtures thereof. Other useful polymers include both natural and synthetic polymers containing at least one catechol group. Examples of these types of polymers are exemplified in the publications listed below.

The substituent on the substituted groups listed above is not limited. Examples of substituents on the catechol and salicylic acid include the following groups amino, alkyl amino, N-substituted amino, -O-alkyl, -O-aryl, alkyl, alkenyl, alkynyl, cycloalkyl, heterocycloalkyl, aryl, heteroaryl, alkylaryl, alkyl-substituted cycloalkyl, alkyl-substituted heterocycloalkyl, araalkyl, alkylheteroaryl, heteroarylalkyl. The substituents listed above may also be oligomers and polymers. The substituent oligomers and polymers may be dopamine based such as mPEG-DOPA oligomers and polymers. The substituent oligomers and polymers may be a polyalkyl glycol such as PEG. Preferably, a PEG-DOPA polymer substituted catachol compound can be used to functionalize the pigment.

Additional useful substituents and substituted catachol compounds may be found in Seng, et al., J. Am. Chem. Soc., 2006, 128, 10676-10677; T. Rajh, et al., J. Phys. Chem. B 2002, 106, 10543-10552 ; J. Dalsin, et al., Langmuir 2005, 21, 640-646; C. Xu, et al., J. Am. Chem. Soc. 2004, 126, 9938-9939; X. Fan, et al., Composite Science and Technology 66 (2006) 1195-1201; J. Dalsin, et al., Materialstoday, September 2005, 38-46; H. Lee, et al., PNAS, August 29, 2006, Vol. 103, No. 35, 12999-13003; G. Westwood, et al., Macromolecules, Published on Web 05/04/2007; B. P. Lee, et al., Macromolecules, 2006, 39, 1740-1748; P. Podsiadlo, et al., Advanced Materials, 19, 7, April 2007, 949-955; H. Gu, J. Am. Chem. Soc., 2005, 127, 34-35; M. Yu, et al., Macromolecules. (1998), 31, 4739-4745; M. Yu, et al., J. Am. Chem. Soc., 1999, 121, 5825-5826. The contents of the above publications are herein incorporated by reference.

Ligating compounds may be added in amounts that would correspond to the formation of at least a monolayer on the metal-oxide layered pigment surface. Typically, the ligating compounds are added at a concentration of about 0.001 to about 5 weight % based on the metal-oxide layered pigment. More typically, the ligating compounds are added at a concentration of about 0.01 to about 1 weight % based on the metal-oxide layered pigment.

Specific examples of ligands include dihydroxy cyclobutenedione, ascorbic acid, catechol, methyl catechol, t-butyl catechol, dopamine, alizarin, polyethylene glycol/dihydroxyphenylalanine copolymer, aminopropyltriethyoxysilane, ammoniumpropyl triethoxysilane, trimethylammoniumpropyl triethoxysilane, examples listed in the publications cited above and mixtures thereof.

The at least one film-former (b), at least one stabilizer (c) and at least one auxiliary ingredient (d) of the disclosed composition varies depending upon the application. These materials can include one or more anionic compounds, non-ionic compounds, amphoteric compounds, zwitterionic compounds, proteins, viscosity modifiers, cationic polymers, anionic polymers, a polyacrylate, a polymethacrylate, a polyacrylate copolymer, a polymethacrylate copolymer, a polyamide, polyaminoamide, polyester, silicone resins, polysaccharides, a silicone fluid, a polyacrylamide, a starch, a gum and mixtures thereof. The at least one film-former, at least one stabilizer and at least one auxiliary ingredient may each be added at a concentration of about 0.05 to about 15 weight % based on the weight of the cosmetic composition. Typically, the at least one film-former, at least one stabilizer and at least one auxiliary ingredient are each added at a concentration of about 0.1 to about 10 weight % based on the weight of the cosmetic composition. The film-former helps the metal oxide pigment stays on the hair and the stabilizer helps to disperse the metal-oxide pigment.

The at least one polymer (e) of the disclosed composition varies depending upon the application. These materials can include one or more anionic surfactants, non-ionic surfactants, amphoteric surfactants, zwitterionic surfactants, proteins, viscosity modifiers, cationic polymers, a polyacrylate, a polymethacrylate, a polyacrylate copolymer, a polymethacrylate copolymer, a polyamide, polyaminoamide, polyester, silicone resins, polysaccharides, a silicone fluid, a polyacrylamide, a starch, a gum and mixtures thereof. The at least one polymer is added at a concentration of about 0.05 to about 15 weight % based on the weight of the cosmetic composition. Typically, the at least one polymer is added at a concentration of about 0.1 to about 10 weight % based on the weight of the cosmetic composition.

Non-limiting examples of anionic compounds include compounds in the classes known as alkyl sulfates, alkyl ether sulfates, alkyl sulfonates, alkyl ether sulfonates, sulfate esters of an alkylphenoxy polyoxyethylene ethanol, alpha-olefin sulfonates, beta alkyloxy alkene sulfonates, alkyl arylsulfonates, alkyl carbonates, alkyl ether carboxylates, fatty acids, succinamates, sulfosuccinates, sarcosinates, octoxynol or nonoxynol phosphates, taurates, fatty taurides, sulfated monoglycerides, fatty acid amino polyoxyethylene sulfactes, isothienates anionic polymers and mixtures thereof. Specific examples of anionic compounds include the ammonim, monoethanolamine, diethanolamine, triethanolamine, isopropylamine, sodium, potassium, lithium, or magnesium salts of lauryl sulfate, dedocylbenzene-sulfonate, lauryl sulfosuccinate, lauryl ether sulfate, lauryl ether carboxylate, lauryl sacrosinate, cocomethyl tauride, and sulfosuccinate half ester amide, anionic polyacrylamides, anionic polyacrylic acids, copolymers of acrylic acid and acrylamide, copolymers containing AMPS and mixtures thereof. The anionic compounds may be added at a concentration of about 0.05 to about 15 weight % based on the weight of the cosmetic composition. Typically, anionic compound is added at a concentration of about 0.1 to about 10 weight % based on the weight of the cosmetic composition.

Non-limiting examples of nonionic compounds includes includes alkoxylated derivatives of the following: fatty alcohols, alkyl phenols, fatty acids, fatty acid esters and fatty acid amides, wherein the alkyl chain is in the C₁₂₋₅₀ range, typically in the C₁₆₋₄₀ range, more typically in the C₂₄ to C₄₀ range, and having from about 1 to about 110 alkoxy groups. The alkoxy groups are selected from the group consisting of C₂-C₆ oxides and their mixtures, with ethylene oxide, propylene oxide, and their mixtures being the typical alkoxides. The alkyl chain may be linear, branched, saturated, or unsaturated. Of these alkoxylated non-ionic compounds, the alkoxylated alcohols are typical, and the ethoxylated alcohols and propoxylated alcohols are more typical. The alkoxylated alcohols may be used alone or in mixtures with those alkoxylated materials disclosed herein-above. The nonionic compounds may be added at a concentration of about 0.05 to about 10 weight % based on the weight of the cosmetic composition. Typically, the nonionic compound is added at a concentration of about 0.1 to about 5 weight % based on the weight of the cosmetic composition.

Other representative examples of such ethoxylated fatty alcohols include laureth-3 (a lauryl ethoxylate having an average degree of ethoxylation of 3), laureth-23 (a lauryl ethoxylate having an average degree of ethoxylation of 23), ceteth-10 (a cetyl alcohol ethoxylate having an average degree of ethoxylation of 10), steareth-10 (a stearyl alcohol ethoxylate having an average degree of ethoxylation of 10), steareth-2 (a stearyl alcohol ethoxylate having an average degree of ethoxylation of 2), steareth-100 (a stearyl alcohol ethoxylate having an average degree of ethoxylation of 100), beheneth-5 (a behenyl alcohol ethoxylate having an average degree of ethoxylation of 5), beheneth-10 (a behenyl alcohol ethoxylate having an average degree of ethoxylation of 10), and other derivatives and mixtures of the preceding.

Commercially available nonionic compounds are Brij^{®} nonionic compounds from Uniqema, Willmington, DE. Typically, Brij^{®} is the condensation products of aliphatic alcohols with from about 1 to about 54 moles of ethylene oxide, the alkyl chain of the alcohol being typically a linear chain and having from about 8 to about 22 carbon atoms, for example, Brij 72 (i.e., Steareth-2) and Brij 76 (i.e., Steareth-10).

Also useful herein as nonionic compounds are alkyl glycosides, which are the condensation products of long chain alcohols, which are the condensation products of long chain alcohols, e.g. C₈-C₃₀ alcohols, with sugar or starch polymers. These compounds can be represented by the formula (S)n --O--R wherein S is a sugar moiety such as glucose, fructose, mannose, galactose, and the like; n is an integer of from about 1 to about 1000, and R is a C₈-C₃₀ alkyl group. Examples of long chain alcohols from which the alkyl group can be derived include decyl alcohol, cetyl alcohol, stearyl alcohol, lauryl alcohol, myristyl alcohol, oleyl alcohol, and the like. More typical examples of these compounds are alkyl polyglucosides wherein S is a glucose moiety, R is a C₈-C₂₀ alkyl group, and n is an integer of from about 1 to about 9. Commercially available examples of these compounds include decyl polyglucoside (available as APG^{®} 325 CS) and lauryl polyglucoside (available as APG^{®} 600CS and 625 CS), all the above-identified polyglucosides APG^{®} are available from Cognis, Ambler, Pa. Also useful herein sucrose ester surfactants such as sucrose cocoate and sucrose laurate.

Other nonionic compounds suitable for use in the present disclosure are glycerly esters and polyglyceryl esters, including but not limited to, glyceryl monesters, typically glycerly monesters of C₁₆-C₂₂ saturated, unsaturated and branched chain fatty acids such as glyceryl oleate, glyceryl monostearate, glyceryl monoisostearate, glyceryl monopalmitate, glyceryl monobehenate, and mixtures thereof, and polyglyceryl esters of C₁₆-C₂₂ saturated, unsaturated and branched chain fatty acids, such as polyglyceryl-4 isostearate, polyglyceryl-3 oleate, polyglyceryl-2 sesquioleate, triglyceryl diisostearate, diglyceryl monooleate, tetraglyceryl monooleate, and mixtures thereof.

Also useful herein as nonionic compounds are sorbitan esters. Preferable are sorbitan esters of C₁₆-C₂₂ saturated, unsaturated and branched chain fatty acids. Because of the manner in which they are typically manufactured, these sorbitan esters usually comprise mixtures of mono-, di-, tri-, etc. esters. Representative examples of suitable sorbitan esters include sorbitan monooleate (e.g., SPAN^{®} 80), sorbitan sesquioleate (e.g., Arlacel^{®} 83 from Uniqema, Wilmington, Del.), sorbitan monoisostearate (e.g., CRILL^{®} 6 from Croda, Inc., Edison, N.J.), sorbitan stearates (e.g., SPAN^{®} 60), sorbitan trioleate (e.g., SPAN^{®} 85), sorbitan tristearate (e.g., SPAN^{®} 65), sorbitan dipalmitates (e.g., SPAN^{®} 40), and sorbitan isostearate. Sorbitan monoisostearate and sorbitan sesquioleate are particularly preferred emulsifiers for use in the present disclosure.

Also suitable for use as nonionic compounds are alkoxylated derivatives of glyceryl esters, sorbitan esters, and alkyl polyglycosides, wherein the alkoxy groups is selected from the group consisting of C₂-C₆ oxides and their mixtures, with ethoxylated or propoxylated derivatives of these materials being ypical. Nonlimiting examples of commercially available ethoxylated materials include TWEEN^{®} (ethoxylated sorbitan mono-, di- and/or tri-esters of C₁₂ to C₁₈ fatty acids with an average degree of ethoxylation of from about 2 to 20).

Non-limiting examples of amphoteric and zwitterionic compounds or surfactants include alkyl, alkyl dimethyl, alkylamido, alkyl amide, alkylamidopropyl, or alkyl dimethylammonium betaine; alky amidopropyl or alkyl sulfobetaine; alkyl, alkylampho, or alkylamphocarboxy glycinate; alkyl, or alkyl substituted imidazoline mono or dicarboxylate; sodium salts of alkyl mono-or dicarboxylates; alkyl beta amino acids; alkyl amidopropyl, or alkyl ether hydroxysultaine; alkyl amidopropyl dimethyl ammonia acetate; alkyl ampho mono-or diacetate; alkyl, or alkyl ampho, or alkyl imino dipropionate; alkyl amphopropionate; alkyl beta amino propionic acid; alkyl dipropionate; alkyl beta iminodipropionate; branched or n-alkyl dimethylamidopropionate; alkyl carboxylated propionate; alkyl, or methyl alkyl imidazoline; fluorinated alkyl amphoteric mixtures; and/or nonionic compounds such as, but not limited to, alkyl, alkyl dimethyl, alkyl amidopropylamine, or bis 2-hydroxy ethyl alkyl amine oxides; alkanolamides; alkyl amides; polyoxyethylene glycol (PEG) of monoglycerides, of sorbitan esters, of branched or linear fatty alcohol ethers, of branched or linear fatty acid ethers, of thioethers; alkyl oxoalcohol PEG; PEG fatty esters; polyoxyethlyene glycol/polyoxpropylene glycol block copolymers; alkyl phenol PEG ethers; alkyl polyglucosides, or polysaccarides, polysiloxane polyethoxylene ether and mixtures thereof. Specific examples include cocamidopropyl betaine, lauramidopropyl betaine, coco/oleamidopropyl betaine, coco betaine, oleyl betaine, cocamidopropyl hydroxysultaine, tallowamidopropyl hydroxysultaine and dihydroxyethyl tallow glycinate and mixtures thereof. The amphoteric and zwitterionic compounds may each be added at a concentration of about 0.05 to about 10 weight % based on the weight of the cosmetic composition. Typically, amphoteric and zwitterionic compounds are each added at a concentration of about 0.1 to about 5 weight % based on the weight of the cosmetic composition.

Non-limiting examples of anionic surfactants include compounds in the classes known as alkyl sulfates, alkyl ether sulfates, alkyl sulfonates, alkyl ether sulfonates, sulfate esters of an alkylphenoxy polyoxyethylene ethanol, alpha-olefin sulfonates, beta alkyloxy alkene sulfonates, alkyl arylsulfonates, alkyl carbonates, alkyl ether carboxylates, fatty acids, succinamates, sulfosuccinates, sarcosinates, octoxynol or nonoxynol phosphates, taurates, fatty taurides, sulfated monoglycerides, fatty acid amino polyoxyethylene sulfactes, isothienates and mixtures thereof. Specific examples of anionic surfactants include the ammonim, monoethanolamine, diethanolamine, triethanolamine, isopropylamine, sodium, potassium, lithium, or magnesium salts of lauryl sulfate, dedocylbenzene-sulfonate, lauryl sulfosuccinate, lauryl ether sulfate, lauryl ether carboxylate, lauryl sacrosinate, cocomethyl tauride, and sulfosuccinate half ester amide and mixtures thereof. The anionic surfactant may be added at a concentration of about 0.05 to about 15 weight % based on the weight of the cosmetic composition. Typically, anionic compound is added at a concentration of about 0.1 to about 10 weight % based on the weight of the cosmetic composition.

Non-limiting examples of nonionic surfactants include alkoxylated derivatives of the following: fatty alcohols, alkyl phenols, fatty acids, fatty acid esters and fatty acid amides, wherein the alkyl chain is in the C₁₂₋₅₀ range, typically in the C₁₆₋₄₀ range, more typically in the C₂₄ to C₄₀ range, and having from about 1 to about 110 alkoxy groups. The alkoxy groups are selected from the group consisting of C₂-C₆ oxides and their mixtures, with ethylene oxide, propylene oxide, and their mixtures being the typical alkoxides. The alkyl chain may be linear, branched, saturated, or unsaturated. Of these alkoxylated non-ionic surfactants, the alkoxylated alcohols are typical, and the ethoxylated alcohols and propoxylated alcohols are more typical. The alkoxylated alcohols may be used alone or in mixtures with those alkoxylated materials disclosed herein-above. The nonionic surfactants may be added at a concentration of about 0.05 to about 10 weight % based on the weight of the cosmetic composition. Typically, nonionic surfactant is added at a concentration of about 0.1 to about 5 weight % based on the weight of the cosmetic composition.

Other representative examples of such ethoxylated fatty alcohols as nonionic surfactants include laureth-3 (a lauryl ethoxylate having an average degree of ethoxylation of 3), laureth-23 (a lauryl ethoxylate having an average degree of ethoxylation of 23), ceteth-10 (a cetyl alcohol ethoxylate having an average degree of ethoxylation of 10), steareth-10 (a stearyl alcohol ethoxylate having an average degree of ethoxylation of 10), steareth-2 (a stearyl alcohol ethoxylate having an average degree of ethoxylation of 2), steareth-100 (a stearyl alcohol ethoxylate having an average degree of ethoxylation of 100), beheneth-5 (a behenyl alcohol ethoxylate having an average degree of ethoxylation of 5), beheneth-10 (a behenyl alcohol ethoxylate having an average degree of ethoxylation of 10), and other derivatives and mixtures of the preceding.

Commercially available nonionic surfactants are Brij® nonionic compounds from Uniqema, Willmington, DE. Typically, Brij® is the condensation products of aliphatic alcohols with from about 1 to about 54 moles of ethylene oxide, the alkyl chain of the alcohol being typically a linear chain and having from about 8 to about 22 carbon atoms, for example, Brij 72 (i.e., Steareth-2) and Brij 76 (i.e., Steareth-10).

Also useful herein as nonionic surfactants are alkyl glycosides, which are the condensation products of long chain alcohols, which are the condensation products of long chain alcohols, e.g. C₈-C₃₀ alcohols, with sugar or starch polymers. These compounds can be represented by the formula (S)n --O--R wherein S is a sugar moiety such as glucose, fructose, mannose, galactose, and the like; n is an integer of from about 1 to about 1000, and R is a C₈-C₃₀ alkyl group. Examples of long chain alcohols from which the alkyl group can be derived include decyl alcohol, cetyl alcohol, stearyl alcohol, lauryl alcohol, myristyl alcohol, oleyl alcohol, and the like. More typical examples of these surfactants are alkyl polyglucosides wherein S is a glucose moiety, R is a C₈-C₂₀ alkyl group, and n is an integer of from about 1 to about 9. Commercially available examples of these surfactants include decyl polyglucoside (available as APG® 325 CS) and lauryl polyglucoside (available as APG® 600CS and 625 CS), all the above-identified polyglucosides APG® are available from Cognis, Ambler, Pa. Also useful herein sucrose ester surfactants such as sucrose cocoate and sucrose laurate.

Other nonionic surfactants suitable for use in the present disclosure are glycerly esters and polyglyceryl esters, including but not limited to, glyceryl monesters, typically glycerly monesters of C₁₆-C₂₂ saturated, unsaturated and branched chain fatty acids such as glyceryl oleate, glyceryl monostearate, glyceryl monoisostearate, glyceryl monopalmitate, glyceryl monobehenate, and mixtures thereof, and polyglyceryl esters of C₁₆-C₂₂ saturated, unsaturated and branched chain fatty acids, such as polyglyceryl-4 isostearate, polyglyceryl-3 oleate, polyglyceryl-2 sesquioleate, triglyceryl diisostearate, diglyceryl monooleate, tetraglyceryl monooleate, and mixtures thereof.

Also useful herein as nonionic surfactants are sorbitan esters. Preferable are sorbitan esters of C₁₆-C₂₂ saturated, unsaturated and branched chain fatty acids. Because of the manner in which they are typically manufactured, these sorbitan esters usually comprise mixtures of mono-, di-, tri-, etc. esters. Representative examples of suitable sorbitan esters include sorbitan monooleate (e.g., SPAN^{®} 80), sorbitan sesquioleate (e.g., Arlacel^{®} 83 from Uniqema, Wilmington, Del.), sorbitan monoisostearate (e.g., CRILL^{®} 6 from Croda, Inc., Edison, N.J.), sorbitan stearates (e.g., SPAN® 60), sorbitan trioleate (e.g., SPAN^{®} 85), sorbitan tristearate (e.g., SPAN® 65), sorbitan dipalmitates (e.g., SPAN® 40), and sorbitan isostearate. Sorbitan monoisostearate and sorbitan sesquioleate are particularly preferred emulsifiers for use in the present disclosure.

Also suitable for use as nonionic surfactants are alkoxylated derivatives of glyceryl esters, sorbitan esters, and alkyl polyglycosides, wherein the alkoxy groups is selected from the group consisting of C₂-C₆ oxides and their mixtures, with ethoxylated or propoxylated derivatives of these materials being ypical. Nonlimiting examples of commercially available ethoxylated materials include TWEEN^{®} (ethoxylated sorbitan mono-, di- and/or tri-esters of C₁₂ to C₁₈ fatty acids with an average degree of ethoxylation of from about 2 to 20).

Non-limiting examples of proteins include collagen, deoxyribonuclease, iodized corn protein, milk protein, protease, serum protein, silk, sweet almond protein, wheat germ protein, wheat protein, alpha and beta helix of keratin proteins, hair proteins, such as intermediate filament proteins, high-sulfur proteins, ultrahigh-sulfur proteins, intermediate filament-associated proteins, high-tyrosine proteins, high-glycine tyrosine proteins, tricohyalin, and mixtures thereof. The protein may be added at a concentration of about 0.05 to about 10 weight % based on the weight of the cosmetic composition. Typically, protein is added at a concentration of about 0.1 to about 5 weight % based on the weight of the cosmetic composition.

Non-limiting examples of viscosity modifiers include water swellable/soluble cationic polymers from quaternized polysaccharides such as trimethyl ammonium substituted epoxide of hydroxyethyl cellulose, diallyldimethyl ammonium salts of hydroxyethylcellulose, deacylated chitin or chitosan, dihydroxypropyl chitosan trimoniurn chloride, hydroxypropltrimethyl ammonium chloride guar, locust bean, or konjac mannan gum; quaternized synthetics such as acrylamide dimethyl diallyl ammonium chloride copolymers, acrylamide/dimethyl diallyl ammonium chloride/acrylic acid terpolymer, quatemized poly (vinyl pyrrolidone/dimethyl amino ethylmethacrylate), poly (vinylpyrrolidone/methacrylamidopropyl trimethylammonium chloride), polyvinyl pyrrolidone/methylvinylimidazolinium chloride or methyl sulfate copolymer, chloroethylether/dimethylaminopropylamine/adipate or azelate terpolymer, vinylpyrrolidone/methacrylamidopropyl trimethylammonium chloride, acrylonitrile/acrylic acid/dimethylpropanediammonium acrylates sulfate terpolymer. Anionic or nonionic polysaccharide polymers such as gum tragacanth, sodium or propylene glycol alginate, kappa-, iota-, or lambda-carrageenan, guar or hydroxyl propyl guar gum, karaya gum, gum Arabic, locust bean gum, konjac mannan gum, gellan, xanthan, succinoglycan or its acidic or enzymatic hydrolysates, sodium carboxymethyl cellulose, methycellulose, hydroxylethylcellulose, hydroxypropylmethylcellulose, and hydroxypropylecellulose; and/or hydrophobically modified anionic, cationic, or nonionic polymers such as, but not limited to, alkyl and/or substituted hydroxyethylcellulose, lauryl dimethyl ammonium substituted epoxide of hydroxyethylcellulose, propoxylated cellulosic, xanthan, succinoglycan, or polygalactomannoses, alkyl methacrylates/crosslinked acrylic acid copolymer and/or acrylonitrile/acrylates block copolymer. The viscosity modifier may be added at a concentration of about 0.05 to about 15 weight % based on the weight of the cosmetic composition. Typically, viscosity modifier is added at a concentration of about 0.1 to about 10 weight % based on the weight of the cosmetic composition.

Typical cationic polymers include, for example, copolymers of vinyl monomers having cationic amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone, and vinyl pyrrolidone. The alkyl and dialkyl substituted monomers preferably have C₁-C₇ alkyl groups, more typically C₁-C₃ alkyl groups. Other suitable comonomers include vinyl esters, vinyl alcohol (made by hydrolysis of polyvinyl acetate), maleic anhydride, propylene glycol, and ethylene glycol. The cationic polymer may be added at a concentration of about 0.05 to about 10 weight % based on the weight of the cosmetic composition. Typically, cationic polymer is added at a concentration of about 0.1 to about 5 weight % based on the weight of the cosmetic composition.

The cationic amines can be primary, secondary, or tertiary amines, depending upon the particular species and the pH of the composition. In general, secondary and tertiary amines, especially tertiary amines, are typical.

Amine-substituted vinyl monomers can be polymerized in the amine form, and then optionally can be converted to ammonium by a quaternization reaction.

Amines can also be similarly quaternized subsequent to formation of the polymer. For example, tertiary amine functionalities can be quaternized by reaction with a salt of the formula R'X wherein R' is a short chain alkyl, preferably a C₁-C₇ alkyl, more preferably a C₁-C₃ alkyl, and X is an anion which forms a water soluble salt with the quaternized ammonium.

Suitable cationic amino and quaternary ammonium monomers include, for example, vinyl compounds substituted with dialkylaminoalkyl acrylate, dialkylaminoalkyl methacrylate, monoalkylaminoalkyl acrylate, monalkylaminoalkyl methacrylate, trialkyl methacryloxyalkyl ammonium salt, trialkyl acryloxyalkyl ammonium salt, diallyl quaternary ammonium salts, and vinyl quaternary ammonium monomers having cyclic cationic nitrogen-containing rings such as pyridinium, imidazolium, and quaternized pyrrolidone, e.g., alkyl vinyl imidazolium, alkyl vinyl pyridinium, alkyl vinyl pyrrolidone salts. The alkyl vinyl portions of these monomers are typically lower alkyls such as the C₁-C₃ alkyls, more preferably C₁ and C₂ alkyls. Suitable amine-substituted vinyl monomers for use herein include dialkylaminoalkyl acrylate, dialkylaminoalkyl methacrylate, dialkylaminoalkyl acrylamide, and dialkylaminoalkyl methacrylamide, wherein the alkyl groups are typically C₁-C₇ hydrocarbyls, more typically C₁-C₃ alkyls.

Non-limiting examples of cationic polymers include polyquaternium 4, polyquaternium 6, polyquaternium 7, polyquaternium 10, polyquaternium 11, polyquaternium 16, polyquaternium 22 and polyquaternium 32.

Other non-limiting polymers include polyacrylates, polymethacrylates, polyacrylate copolymers, polymethacrylate copolymers, polyamines polyaminoamide, polyester, silicone fluids, and polysaccharides. These polymers may be anionic, cationic or nonionic.. The polyacrylate and polymethacrylate copolymers can be copolymers of acrylate and methacrylate with any suitable monomer such as acrylamide, DADMAC, vinylsulfonic acid, styrenesulfonic acid, naphthalenesulfonic acid, acrylamidoalkylsulfonic acid, esters of acrylic and methacrylic acid, vinyl ethers, vinyl esters, vinylprrolidone and salts of the sulfonic acids listed above.

The polyesters may be obtained, in a known way, by polycondensation of a aliphatic and aromatic diacids with aliphatic and aromatic diols and with polyols. Use may be made, as aliphatic diacids, of succinic acid, glutaric acid, adipic acid, pimelic acid, suberic acid and sebacic acid. Use may be made, as aromatic diacids, of terephthalic acid and isophthalic acid, and of derivatives, such as phthalic anhydride. Use may be made, as aliphatic diols, of ethylene glycol, propylene glycol, diethylene glycol, neopentyl glycol, cyclohexanedimethanol and 4,4'-(1-methylpropylidene)bisphenol. Use may be made, as polyols, of glycerol, pentaerythritol, sorbitol and trimethylolpropane. The polyester may be added at a concentration of about 0.05 to about 10 weight % based on the weight of the cosmetic composition. Typically, polyester is added at a concentration of about 0.1 to about 5 weight % based on the weight of the cosmetic composition.

Non-limiting examples of silicone fluids include polyalkyl siloxanes, polyarylsiloxanes, polyarylalkyl siloxanes, polyether siloxane copolymers and mixtures thereof.

The polyalkylsiloxane fluids that may be used include, for example, polydimethylsiloxanes. These siloxanes are available, for example, from the General Electric Company in their Viscasil R and SF 96 series, and from Dow Corning in their Dow Corning 200 series.

The polyalkylaryl siloxane fluids that may be used, also include, for example, polymethylphenylsiloxanes. These siloxanes are available, for example, from the General Electric Company as SF 1075 methyl phenyl fluid or from Dow Corning as 556 Cosmetic Grade Fluid.

The polyether siloxane copolymers that may be used include, for example, a polypropylene oxide modified polydimethylsiloxane (e.g., Dow Corning DC-1248) although ethylene oxide or mixtures of ethylene oxide and propylene oxide may also be used. For water insoluble silicones the ethylene oxide and polypropylene oxide level must be sufficiently low to prevent solubility in water and the composition hereof.

Non-limiting examples of polysaccharides include those selected from cellulose, and cellulose derivatives. Typical cellulose derivatives include alkyl hydroxyalkyl cellulose ethers that are given the CTFA designation cetyl hydroxyethylcellulose, which is the ether of cetyl alcohol and hydroxyethylcellulose, sold under the tradename NATROSEL^{®} CS PLUS from Aqualon Corporation. Other useful polysaccharides include scleroglucans which are a linear chain of (1-3) linked glucose units with a (1-6) linked glucose every three units, a commercially available example of which is CLEAROGEL^{™} CS 11 from Michel Mercier Products Inc.

Starches and modified starches are also useful polymers in the disclosed composition. Acrylate modified starches such as WATERLOCK^{®} from Grain Process Corporation may be used. Hydroxypropyl starch phosphate, tradename STRUCTURE XL from National Starch is another example of a useful modified starch, and other useful examples include ARISTOFLEX HMB (Ammonium Acrylodimethyltaruate/Beheneth-25 Methacrylate Crosspolymer) from Clariant and Cationic stabylens.

The molecular weight of the polymers described in this specification varies depending upon the polymer type and application. However, the polymer molecular weight varies from about 3,000 to about 10 million g/mol. Typically, the molecular weight is from about 5,000 to about 1,000,000 g/mol, more typically from about 10,000 to about 500,000 g/mol and even more typically from about 10,000 to 200,000 g/mol. Unless otherwise noted, the polymers described in this specification may be added at a concentration of about 0.05 to about 10 weight % based on the weight of the cosmetic composition. Typically, the polymers described in this specification when added are added at a concentration of about 0.1 to about 5 weight % based on the weight of the cosmetic composition.

Other auxiliary ingredients of the disclosed composition can vary depending upon the application. The auxiliary ingredient can include one or more selected from a fatty carboxylic acid and its salt, a fatty ether carboxylic acid and its salt, a fatty phosphoric acid and its salt, a fatty ether carboxylic acid and its salt, a polyamine, an oil, a fatty ester, a hydrocarbon, a silicone, a wax, a fatty alcohol, an antibacterial agent, a sunscreen, a preservative, a PH adjusting agent, a bleaching agent, a perfume, a filler, a thickening agent, a sequestering agent, a fragrance, a coloring agent or dye, a film forming agent, an amino acid, a cationic conditioner, a water insoluble ingredient.

Non-limiting examples of fatty carboxylic acids includes fatty acids having about 6 to 40 carbon atoms corresponding formula (I)

RCOOH (I)

wherein:
R is a hydrocarbon radical containing from about 6 to about 40 carbon atoms. In addition, R is linear or branched, acyclic or cyclic, saturated or unsaturated, aliphatic or aromatic, substituted or unsubstituted. Typically, R is a linear or branched, acyclic C₆₋₄₀ alkyl or alkenyl group or a C₁₋₄₀ alkyl phenyl group, more typically a C₈₋₂₂ alkyl or alkenyl group or a C₄₋₁₈ alkyl phenyl group, and even more typically a C₁₂₋₁₈ alkyl group or alkenyl group or a C₆₋₁₆ alkyl phenyl group.

Suitable fatty acids having from about 6 to about 40 carbon atoms include, but are not limited to the following representatives referred to by their INCI names (INCI: nomenclature for raw materials according to the International Cosmetic Ingredient Dictionary, 10^{th} Edition, published by the Cosmetic, Toiletry and Fragrance Association Inc. (CTFA), Washington D.C., USA): Arachidic Acid, Arachidonic Acid, Beeswax Acid, Capric Acid, Caproic Acid, Caprylic Acid, Coconut Acid, Isostearic Acid, Lauric Acid, Linoleic Acid, Linolenic Acid, Myristic Acid, Oleic Acid, Olive Acid, Palmitic Acid, Rapeseed Acid, Stearic Acid, Tallow Acid, Undecanoic Acid, Undecylenic Acid or Wheat Germ Acid and mixtures thereof.

Typical fatty acids having from about 6 to about 40 carbon atoms include Capric Acid, Caprylic Acid, Lauric Acid, Oleic Acid, Isostearic Acid, and Stearic Acid.

Non-limiting examples of fatty ether carboxylic acid includes compounds corresponding to formula (II):

RO[CH₂O]ᵤ[(CH₂)ₓCH(R') (CH₂)_{y} (CH₂)_{z} O]ᵥ[CH₂CH₂ O]_{w}CH₂COOH (II)

wherein:
R is a hydrocarbon radical containing from about 6 to about 40 carbon atoms;
u, v and w, independently of one another, represent numbers of from 0 to 60;
x, y and z, independently of one another, represent numbers of from 0 to 13;
R' represents hydrogen, alkyl, and
the sum of x+y+z is ≥ 0;

Ether carboxylic acids corresponding to formula (II) can be obtained by alkoxylation of alcohols ROH with ethylene oxide as the sole alkoxide or with several alkoxides and subsequent oxidation. The numbers u, v, and w each represent the degree of alkoxylation. Whereas, on a molecular level, the numbers u, v and w and the total degree of alkoxylation can only be integers, including zero, on a macroscopic level they are mean values in the form of broken numbers.

In formula (II), R is linear or branched, acyclic or cyclic, saturated or unsaturated, aliphatic or aromatic, substituted or unsubstituted. Typically, R is a linear or branched, acyclic C₆₋₄₀ alkyl or alkenyl group or a C₁₋₄₀ alkyl phenyl group, more typically a C₈₋₂₂ alkyl or alkenyl group or a C₄₋₁₈ alkyl phenyl group, and even more typically a C₁₂₋₁₈ alkyl group or alkenyl group or a C₆₋₁₆ alkyl phenyl group; u, v, w, independently of one another, is typically a number from 2 to 20, more typically a number from 3 to 17 and most typically a number from 5 to 15; x, y, z, independently of one another, is typically a number from 2 to 13, more typically a number from 1 to 10 and most typically a number from 0 to 8;

Suitable ether carboxylic acids or ether carboxylates include, but are not limited to, the following representatives referred to by their INCI names (INCI: nomenclature for raw materials according to the International Cosmetic Ingredient Dictionary, 7^{th} Edition, published by the Cosmetic, Toiletry and Fragrance Association Inc. (CTFA), Washington D.C., USA): Butoxynol-5 Carboxylic Acid, Butoxynol-19 Carboxylic Acid, Capryleth-4 Carboxylic Acid, Capryleth-6 Carboxylic Acid, Capryleth-9 Carboxylic Acid, Ceteareth-25 Carboxylic Acid, Coceth-7 Carboxylic Acid, C₉₋₁₁ Pareth-6 Carboxylic Acid, C₁₁₋₁₅ Pareth-7 Carboxylic Acid, C₁₂₋₁₃ Pareth-5 Carboxylic Acid, C₁₂₋₁₃ Pareth-8 Carboxylic Acid, C₁₂₋₁₃ Pareth-12 Carboxylic Acid, C₁₂₋₁₅ Pareth-7 Carboxylic Acid, C₁₂₋₁₅ Pareth-8 Carboxylic Acid, C₁₄₋₁₅ Pareth-8 Carboxylic Acid, Deceth-7 Carboxylic Acid, Laureth-3 Carboxylic Acid, Laureth-4 Carboxylic Acid, Laureth-5 Carboxylic Acid, Laureth-6 Carboxylic Acid, Laureth-8 Carboxylic Acid, Laureth-10 Carboxylic Acid, Laureth-11 Carboxylic Acid, Laureth-12 Carboxylic Acid, Laureth-13 Carboxylic Acid, Laureth-14 Carboxylic Acid, Laureth-17 Carboxylic Acid, PPG-6-Laureth-6 Carboxylic Acid, PPG-8-Steareth-7 Carboxylic Acid, Myreth-3 Carboxylic Acid, Myreth-5 Carboxylic Acid, Nonoxynol-5 Carboxylic Acid, Nonoxynol-8 Carboxylic Acid, Nonoxynol-10 Carboxylic Acid, Octeth-3 Carboxylic Acid, Octoxynol-20 Carboxylic Acid, Oleth-3 Carboxylic Acid, Oleth-6 Carboxylic Acid, Oleth-10 Carboxylic Acid, PPG-3-Deceth-2 Carboxylic Acid, Capryleth-2 Carboxylic Acid, Ceteth-13 Carboxylic Acid, Deceth-2 Carboxylic Acid, Hexeth-4 Carboxylic Acid, Isosteareth-6 Carboxylic Acid, Isosteareth-11 Carboxylic Acid, Trudeceth-3 Carboxylic Acid, Trideceth-6 Carboxylic Acid, Trideceth-8 Carboxylic Acid, Trideceth-12 Carboxylic Acid, Trideceth-3 Carboxylic Acid, Trideceth-4 Carboxylic Acid, Trideceth-7 Carboxylic Acid, Trideceth-15 Carboxylic Acid, Trideceth-19 Carboxylic Acid, Undeceth-5 Carboxylic Acid and mixtures thereof.

Typical Carboxylic Acids are Oleth-10 Carboxylic Acid, Laureth-5 Carboxylic Acid and Laureth-11 Carboxylic Acid.

Non-limiting examples of fatty phosphoric acids include compounds corresponding to Formula II:

R-O-P(O)(OH)₂ (III)

wherein:
R is a hydrocarbon radical containing from about 6 to about 40 carbon atoms. In addition, R is linear or branched, acyclic or cyclic, saturated or unsaturated, aliphatic or aromatic, substituted or unsubstituted. Typically, R is a linear or branched acyclic C₆₋₄₀ alkyl or alkenyl group or a C₁₋₄₀ alkyl phenyl group, more typically a C₈₋₂₂ alkyl or alkenyl group or a C₄₋₁₈ alkyl phenyl group and most typically a C₁₂₋₁₈ alkyl group or alkenyl group or a C₆₋₁₆ alkyl phenyl group.

Typical fatty phosphoric acids include capryl phosphate, caprylyl phosphate, lauryl phosphate, oleyl phosphate, isostearyl phosphate, stearyl phosphate and cetyl phosphate.

Non-limiting examples of fatty ether phosphoric acids compounds corresponding to formulas IV and V:

RO[CH₂O]ᵤ[(CH₂)ₓCH(R')(CH₂)_{y}(CH₂)_{z}O]ᵥ[CH₂CH₂O]_{w}-PO-(OH)₂

Formula IV,

{RO[CH₂O]ᵤ[(CH₂)ₓCH(R')(CH₂)_{y}(CH₂)_{z}O]ᵥ[CH₂CH₂O]_{w}}₂PO-(OH)

Formula V
and combinations thereof,
wherein:
R is a hydrocarbon radical containing from about 6 to about 40 carbon atoms;
u, v and w, independently of one another, represent numbers of from 0 to 60;
x, y and z, independently of one another, represent numbers of from 0 to 13;
R' represents hydrogen, alkyl, and
the sum of x+y+z being ≥ 0.

The numbers u, v, and w each represent the degree of alkoxylation. Whereas, on a molecular level, the numbers u, v and w and the total degree of alkoxylation can only be integers, including zero, on a macroscopic level they are mean values in the form of broken numbers.

In formulas IV and V, R is linear of branched, acyclic or cyclic, saturated or unsaturated, aliphatic or aromatic, substituted or unsubstituted, typically a linear or branched, acyclic C₆₋₄₀ alkyl or alkenyl group or a C₁₋₄₀ alkyl phenyl group, more typically a C₈₋₂₂ alkyl or alkenyl group or a C₄₋₁₈ alkyl phenyl group, even more typically a C₁₂₋₁₈ alkyl group or alkenyl group or a C₆₋₁₆ alkyl phenyl group; u, v, w, independently of one another, is typically a number from 2 to 20, more typically a number from 3 to 17 and most typically a number from 5 to 15; x, y, z, independently of one another, is typically a number from 2 to 13, more typically a number from 1 to 10 and most typically a number from 0 to 8.

Typical fatty ether phosphoric acids include PPG-5-Ceteth-10 phosphate (CRODAFOS SG®), Oleth-3 phosphate (CRODAFOS N3 acid), Oleth-10 phosphate (CRODAFOS N10 acid), and a mixture of Ceteth-10 phosphate and Dicetyl phosphate (CRODAFOS CES) all sold by Croda.

Non-limiting examples of fatty acids are the same as those described above for the at least one fatty acid described above. This includes carboxylate salts of the fatty acids listed above. The sodium, potassium, ammonium, calcium and magnesium carboxylates of the fatty acids listed above are typical examples of the carboxylate salts of the fatty acids.

The polyamine may, for example, be chosen from a polyethyleneimine, a polyvinylamine, an aminated polysaccharide, an amine substituted polyalkylene glycol, an amine substituted polyacrylate crosspolymer, an amine substituted polyacrylate, an amine substituted polymethacrylate, a protein, an amine substituted polyester, a polyamino acid, an amodimethicone, a polyalkylamine, diethylene triamine, triethylenetetramine, spermidine, spermine and mixtures thereof. The molecular weight of the polyamine described in this specification varies depending upon the polymer type and application. However, the polyamine molecular weight varies from about 3,000 to about 10 million g/mol. Typically, the molecular weight is from about 5,000 to about 1,000,000 g/mol, more typically from about 10,000 to about 500,000 g/mol and even more typically from about 10,000 to 200,000 g/mol. Unless otherwise noted, the polyamine described in this specification may be added at a concentration of about 0.05 to about 10 weight % based on the weight of the cosmetic composition. Typically, the polyamine described in this specification when added are added at a concentration of about 0.1 to about 5 weight % based on the weight of the cosmetic composition.

Non-limiting examples of polyethyleneimine include Lupasol^{™} products commercially available from BASF. Suitable examples of Lupasol^{™} polyethyleneimines include Lupasol^{™} PS, Lupasol^{™} PL, Lupasol^{™} PR8515, Lupasol^{™} G20, Lupasol^{™} G35 as well as Lupasol^{™} SC^{®} Polyethyleneimine Reaction Products (such as Lupasol^{™} SC-61B, Lupasol^{™} SC-62J^{®}, and Lupasol^{™} SC-86X^{®}). Other non-limiting examples of polyethyleneimines which may be used in the composition according to the present disclosure are the Epomin^{™} products commercially available from Aceto. Suitable examples of Epomin^{™} polyethyleneimines include Epomin^{™} SP-006, Epomin^{™} SP-012, Epomin^{™} SP-018, and Epomin^{™} P-1000. These examples include substituted polyethyleneimines.

Non-limiting examples of polyvinylamines include Lupamines^{®} 9095, 9030, 9010, 5095 and 1595 from BASF.

An example of an amine substituted polyalkylene glycol includes PEG-15 cocoloryamine from Cognis.

In another embodiment, the polyamine compound is chosen from proteins and protein derivatives. Non-limiting examples of suitable proteins and protein derivatives for use in the present disclosure include those listed at pages 1701 to 1703 of the C.T.F.A. International Cosmetic Ingredient Dictionary and Handbook, 8th edition, vol. 2, (2000) (incorporated herein by reference). In one embodiment, the at least one polyamine compound is chosen from wheat protein, soy protein, oat protein, collagen, and keratin protein.

In another embodiment, the polyamine compound is chosen from compounds comprising lysine, compounds comprising arginine, compounds comprising histidine, and compounds comprising hydroxylysine. Not limiting examples include chitosan and polylysine.

An example of an amine substituted polyacrylate crosspolymer includes Carbopol^{®} Aqua CC polymer from Noveon, Inc.

Non-limiting examples of oils include plant oil such as olive oil, avocado oil, coconut oil, aloe vera oil, almond oil, castor oil, jojoba oil, peanut oil, sesame oil, hazelnut oil, sunflower oil, colza oil, grapeseed oil, linseed oil and palm oil. Other types of oils include ester oils such as cetyl octanoate, octyl isonanoanate, myristyl lactate, cetyl lactate, isopropyl myristate, myristyl myristate, isopropyl palmitate, isopropyl adipate, butyl stearate, decyl oleate, cholesterol isostearate, glycerol monostearate, glycerol distearate, glycerol tristearate, alkyl lactate, alkyl citrate and alkyl tartrate, sucrose ester and sorbitol ester.

Non-limiting examples of hydrocarbon oils include mineral oil, petrolatum, paraffins, iso-paraffins, aromatic hydrocarbons and C₁₀₋₄₀ hydrocarbons which may be aliphatic, aromatic, arylaliphatic or mixtures thereof and the aliphatic hydrocarbons may be straight chain, branched, cyclic or combinations thereof.

Non-limiting examples of silicones include phenyltrimethicone, dimethicone, cyclomethicone, dimethicone copolyol, aminosilicone, laurylmethicone copolyol, cetyl dimethicone, cetyl triethylammonium dimethicone' copolyol phthalate, dimethicone copolyol lactate, silicone quaternium 13, stearalkonium dimethicone copolyol phthalate, stearaminopropyl dimethicone and polyorganosiloxanes such as polydimethylsiloxane.

Non-limiting examples of waxes include paraffin wax, beeswax, candelilla wax, carnauba wax, jasmine wax, jojoba wax and mimosa wax.

Non-limiting example of fatty alcohols include compounds of formula (VI):

R-OH (VI)

where R is as described above for the at least one fatty acid.

Non-limiting fatty esters include esters formed from the fatty acid of formula (I) and C₁₋₁₀ alcohols and esters formed from the fatty alcohol of formula VI and C₁₋₁₀ carboxylic acids.

In addition, non-limiting specific examples of water-insoluble ingredients includes isopropyl palmitate, capric triglyceride, caprylic triglyceride, isodecane, polylsobutylene, Vitamin E, Vitamin E Acetate, Vitamin A, Vitamin A Palmitate, 2-oleamido-1,3-octadecanediol, octymethoxy cinnamate, octyl salicylate and mixtures thereof.

Non-limiting examples of amino acids include amino acids derived from the hydrolysis of various proteins as well as the salts, esters, and acyl derivatives thereof. Non-limiting examples of such amino acid agents include amphoteric amino acids such as alkylamido alkylamines, i.e. stearyl acetyl glutamate, capryloyl silk amino acid, capryloyl collagen amino acids, capryloyl keratin amino acids, capryloyl pea amino acids, cocodimonium hydroxypropyl silk amino acids, corn gluten amino acids, cysteine, glutamic acid, glycine, hair keratin amino acids, amino acids such as asparatic acid, threonine, serine, glutamic acid, proline, glycine, alanine, cystine, valine, methionine, isoleucine, leucine, tyrosine, phenylalanine, cysteic acid, lysine, histidine, arginine, cysteine, tryptophan, citrulline, lysine, silk amino acids, wheat amino acids and mixtures thereof.

Non-limiting examples of cationic conditioners include quaternium 27, behenamidopropyl PG-dimonium chloride, hydroxyethyl tallowdimonium chloride, hexadimethrine chloride, stearalkonium chloride and cetrimonium chloride.

Non-limiting examples of antibacterial agents include bacitracin, phenol, benzethonium chloride, erythromycin, neomycin, tetracycline, chlortetracycline and mixtures thereof.

Non-limiting examples of sunscreens include benzophenones, bornelone, butyl paba, cinnamidopropyl trimethyl ammonium chloride, disodium distryrylbiphenyl disulfonate, paba, potassium methoxycinnamate, butyl methoxydibenzoylmethane, octyl methoxycinnamate, oxybenzone, octocrylene, octyl salicylate, phenylbenzimidazole sulfonic acid, ethyl hydroxypropyl aminobenzoate, menthyl anthranilate, aminobenzoic acid, cinoxate, diethanolamine methoxycinnamate, glyceryl aminobenzoate, titanium dioxide, zinc oxide, oxybenzone, Padimate O, red petrolatum, and mixtures thereof.

Non-limiting examples of preservatives include ethanol, polyvinyl alcohol, phenoxyethanol, benzyl alcohol, methyl paraben, propyl paraben and mixtures thereof.

Non-limiting examples of PH adjusting agents includes potassium acetate, sodium carbonate, sodium hydroxide, phosphoric acid, succinic acid, sodium citrate, citric acid, boric acid, lactic acid, sodium hydrogen carbonate and mixtures thereof.

Bleaching agents include, but not limited to, hydrogen peroxide, perborate and persufate salts. EDTA and other aminocarboxylates may be used as sequestering agents. Anti-dandruff agents such as zinc pyrithione, salicylic acid, climbazole, ketoconazole, sulfur piroctone olamine, selenium sulfide and mixtures thereof may also be used as an auxiliary ingredient.

The coloring agents may be white or colored, and mineral and/or organic pigments. Among the mineral pigments that may be mentioned are titanium dioxide, optionally surface-treated, zirconium oxide and cerium oxide, and also zinc oxide, iron oxide (black, yellow or red), chromium oxide, manganese violet, ultramarine blue, chromium hydrate and ferric blue, and metal powders, for instance aluminium powder or copper powder, and mixtures thereof.

Among the organic pigments that may be mentioned are carbon black, pigments of D & C type, and lakes based on cochineal carmine or on barium, strontium, calcium or aluminium, and mixtures thereof.

The coloring agent may also comprise at least one nacre or nacreous pigment.

The nacres may be present in the composition in a proportion of from 0 to 25% (especially 0.01% to 25%) by weight, typically from 0.01% to 15% by weight and more typically from 0.02% to 5% by weight relative to the total weight of the composition.

The nacreous pigments may be chosen from white nacreous pigments such as mica coated with titanium or with bismuth oxychloride, colored nacreous pigments such as titanium mica coated with iron oxides, titanium mica coated especially with ferric blue or with chromium oxide; titanium mica coated with an organic pigment of the abovementioned type, and also nacreous pigments based on bismuth oxychloride, and mixtures thereof.

The composition of the disclosure may, inter alia, comprise dyes that are soluble in physiological medium and in particular liposoluble or water-soluble dyes.

The coloring agent may also comprise a colorant chosen from water-soluble or liposoluble dyes or alternatively coloring polymers. The colorant may be present in the composition in content of coloring active material ranging from 0 to 6% (especially 0.01% to 6%) by weight and typically ranging from 0.01% to 3% by weight relative to the total weight of the composition.

The liposoluble dyes are, for example, soybean oil, Sudan brown, DC Yellow 11, DC Orange 5, quinoline yellow, Sudan Red III (CTFA name D&C red 17), lutein, quinizarine green (CTFA name DC green 6), Alizurol SS purple (CTFA name DC violet No. 2), carotenoid derivatives, for instance lycopene, beta-carotene, bixin or capsanthin, and/or mixtures thereof.

Among the water-soluble dyes that may be mentioned are dyeing plant extracts such as, for example, *Aleurites moluccana* Willd, *Alkanna tinctoria* Tausch, *Areca catechu L., Arrabidaea chica* E. and B., *Bixa orellana* L (annatto), *Butea monosperma* Lam, *Caesalpina echinata* Lam, *Caesalpina sappan L., Calophyllum inophyllum L., Carthamus tinctorius L., Cassia alata L., Chrozophora tinctoria L., Crocus sativus L., Curcuma longa L., Diospyros gilletii* Wild, *Eclipta prostrata L., Gardenia erubescens* Stapf. and Hutch., *Gardenia terniflora* Schum. and Thonn., *Genipa americana L., Genipa brasiliensis L., Guibourtia demeusei* (Harms) J. Leon, *Haematoxylon campechianum L., Helianthus annuus, Humiria balsamifera* (Aubl.) St-Hil., *Isatis tinctoria L., Mercurialis perenis, Monascus purpureus, Monascus ruber, Monascus pilosus, Morus nigra L., Picramnia spruceana, Pterocarpus erinaceus* Poir., *Pterocarpus soyauxii* Taub., *Rocella tinctoria L., Rothmannia whitfieldii* (Lindl.) Dand., *Schlegelia violacea* (Aubl.) Griseb., *Simira tinctoria* Aublet, *Stereospermum kunthianum* Cham., *Symphonia globulifera L., Terminalia catappa L., sorghum, Aronia melanocarpa,* naphthoquinones including lawsone, derived from *Lawsonia inermis* L., also known as henna, or from *Impatiens balsamina,* red wood extracts as described in document WO 98/44902, beetroot juice, the disodium salt of suschin, anthocyans, for instance extracts of red berries, dihydroxyacetone, monocarbonyl or polycarbonyl derivatives such as isatin, alloxan, ninhydrin, glyceraldehyde, mesotartaric aldehyde, pyrazoline-4,5-dione derivatives, and mixtures thereof, these skin-coloring agents optionally being combined with direct dyes or indole derivatives, and/or mixtures thereof.

These dyeing plant extracts may be in the form of a lyophilizate, a paste or a solution: generally, the leaves of the dyeing plant are ground to obtain a powder. This powder is dissolved in an aqueous phase for several hours. The mixture is subsequently centrifuged and then filtered. The filtrate obtained is frozen and then lyophilized.

The coloring agent may also comprise a coloring polymer, ie a polymer comprising at least one organic coloring group. The coloring polymer generally contains less than 10% by weight of colorant relative to the total weight of the polymer.

The coloring polymer may be of any chemical nature, especially a polyester, polyamide, polyurethane, polyacrylic, poly(meth)acrylic, polycarbonate, polymers of natural origin, for instance cellulose polymers or chitosan polymers, or mixtures thereof, and preferably polyester or polyurethane polymers.

The coloring polymer may comprise a coloring group [lacuna] may be grafted, especially by covalent bonding, onto the polymer chain, as described in documents WO-A-96/29046, WO-A-92/01022, WO-A-90/07558 and BE-A-609 054.

In particular, the coloring polymer may be a copolymer based on at least two different monomers, at least one of which is an organic coloring monomer.

The monomers of the coloring polymer may be chosen from anthraquinones, methines, bis-methines, aza-methines, arylidenes, 3H-dibenzo[7,i-j]isoquinolines, 2,5-diarylaminoterephthalic acids and esters thereof, phthaloylphenothiazines, phthaloylphenoxazines, phthaloylacridone, anthrapyrimidines, anthrapyrazoles, phthalocyanins, quinophthalones, indophenols, perinones, nitroarylamines, benzodifurans, 2H-1-benzopyran-2-ones, quinophthalones, perylenes, quinacridones, triphenodioxazines, fluoridines, 4-amino-1,8-naphthalimides, thioxanthrones, benzanthrones, indanthrones, indigos, thioindigos, xanthenes, acridines, azines and oxazines.

Coloring monomers are described especially in documents U.S. Pat. No. 4,267,306; U.S. Pat. No. 4,359,570; U.S. Pat. No. 4,403,092, U.S. Pat. No. 4,617,373; U.S. Pat. No. 4,080,355; U.S. Pat. No. 4,740,581; U.S. Pat. No. 4,116,923; U.S. Pat. No. 4,745,173; U.S. Pat. No. 4,804,719; U.S. Pat. No. 5,194,463; U.S. Pat. No. 5,804,719; WO-A-92/07913.

Polymeric colorants are described especially in documents U.S. Pat. No. 4,804,719; U.S. Pat. No. 5,032,670; U.S. Pat. No. 4,999,418; U.S. Pat. No. 5,106,942; U.S. Pat. No. 5,030,708; U.S. Pat. No. 5,102,980; U.S. Pat. No. 5,043,376; U.S. Pat. No. 5,194,463; WO-A-92/07913; WO-A-97/24102, the content of which is incorporated into the present patent application by reference.

Sulphopolyester coloring-polymers such as those described in document WO-A-97/24102 are typically used.

The coloring polymers may be present in the composition according to the disclosure in a content ranging from 0% to 50% by weight (0.01% to 50%), typically ranging from 0.5% to 25% by weight and better still ranging from 0.2% to 20% by weight relative to the total weight of the composition.

According to the disclosure, the goniochromatic pigments and the additional coloring agent are present in the composition according to the disclosure in a weight ratio of goniochromatic pigments to active material of the additional coloring agent of less than or equal to 2, typically ranging from 0.1 to 1.5 and even more typically ranging from 0.5 to 1.5, for example equal to about 1 (i.e. ranging from 0.9 to 1.1).

In addition to the additional coloring agent, the composition according to the disclosure may also contain fillers. The term "fillers" should be understood as meaning colorless or white, mineral or synthetic particles of any form, which are insoluble in the medium of the composition irrespective of the temperature at which the composition is manufactured. These fillers serve especially to modify the rheology or texture of the composition.

The fillers may be mineral or organic of any form, platelet-shaped, spherical or oblong-shaped, irrespective of the crystallographic form (for example leaflet, cubic, hexagonal, orthorhombic, etc.). Mention may be made of talc, mica, silica, kaolin, polyamide powder (NylonOrgasol^{®} from Atochem), poly-beta-alanine powder and polyethylene powder, tetrafluoroethylene polymer (Teflon^{®} powders, lauroyllysine, starch, boron nitride, polymeric hollow microspheres such as those of polyvinylidene chloride/acrylonitrile, for instance Expancel^{®} (Nobel Industrie), acrylic acid polymers (Polytrap from the company Dow Corning), and silicone resin microbeads (for example Tospearls^{®} from Toshiba), elastomeric polyorganosiloxane particles, precipitated calcium carbonate, magnesium carbonate and magnesium hydrocarbonate, hydroxyapatite, hollow silica microspheres (Silica Beads^{®} from Maprecos), glass microcapsules and ceramic microcapsules; metal soaps derived from organic carboxylic acids containing from 8 to 22 carbon atoms and typically from 12 to 18 carbon atoms, such as, for example, zinc, magnesium or lithium stearate, zinc laurate or magnesium myristate. The fillers may be present in a proportion of from 0 to 90% by weight, typically 0.01% to 50% by weight and more typically from 0.02% to 30% by weight relative to the total weight of the composition.

The film-former agents comprise, for example, film-forming polymers.

As used herein, the term "film-forming polymer" means a polymer capable of forming, by itself alone or in the presence of an additional agent which is able to form a film, an isolable film, for example, a film which is continuous and which adheres to a support, such as to keratinous substances.

In the composition, use may be made of a single film-forming polymer or of a blend of film-forming polymers. This film-forming polymer can be chosen for example, from the group comprising radical polymers, polycondensates and polymers of natural origin.

The film-forming polymer can be organic or inorganic.

The film-forming agent may be film-forming polymers which are soluble or dispersible in at least one category of organic solvents, such as, for example, ketones, alcohols, glycols, propylene glycol ethers, short-chain esters, alkanes and their mixtures.

The corresponding polymers can be of any chemical nature. For example, they can result from homopolymerization and copolymerization of unsaturated monomers, from polycondensation, and from the modification of natural polymers, for example, polysaccharides. The weight-average molecular masses (Mw) of these polymers can range from 3,000 to 1,000,000 g/mol, for example, from 5,000 to 800,000 g/mol and further, for example, from 10.000 to 500,000 g/mol.

The following polymers can, for example, be used among polymers which are soluble or dispersible in organic solvents such as:
(a) (Meth)acrylic acid ester and amide homopolymers and copolymers, such as polymers resulting from the polymerization and copolymerization of methyl, ethyl, propyl, butyl, isobutyl, tert-butyl, pentyl, hexyl, cyclohexyl, 2-ethylhexyl, heptyl, octyl, isobornyl, norbornyl and adamantyl acrylates and methacrylates and the corresponding (meth)acrylamides. These polymers can, for example, comprise from 0 to 20% of a polar comonomer, such as (meth)acrylic acid, (meth)acrylamide, hydroxyethyl (meth)acrylate, 2-hydroxypropyl (meth)acrylate and (meth)acrylonitrile. They can also result from copolymerization with styrenes and substituted styrenes.
(b) Vinyl ester and amide homopolymers and copolymers, such as homopolymers and copolymers resulting from the polymerization of vinyl acetate, vinyl propionate and vinyl versatate, optionally with the presence of at least one polar comonomer, such as crotonic acid, allyloxyacetic acid, maleic anhydride (or acid), itaconic anhydride (and acid), vinylacetamide and vinylformamide. Likewise, they can result from the copolymerization of at least one of the monomers mentioned with styrenes and substituted styrenes.
(c) Celluloses and cellulose derivatives, such as nitrocelluloses and cellulose esters, for example cellulose acetates, cellulose propionates, cellulose butyrates, cellulose acetate propionates and cellulose acetate butyrates.
(d) Polycondensates which are soluble or dispersible in these solvents. They are generally used as main film-forming agent or else as co-film-forming agents for one of the categories of polymers mentioned above (a to c), for example, if they are of low molecular weight (Mw<20,000 g/mol). They can be chosen from the following polymers and copolymers: polyurethanes, acrylic polyurethanes, polyureas, polyurea polyurethanes, polyester polyurethanes, polyether polyurethanes, polyesters, polyesteramides, polyesters with a fatty chain, epoxys and arylsulphonamide condensates, such as tosylamide/formaldehyde condensates.

Non-limiting mention may be made, among these polycondensates, for example, if they are used as film-forming agent or co-film-formnig agent for at least one nitrocellulose and for a cellulose ester (category c), of:

Polyesters, for example, polyesters with a fatty chain such as copolymers with the CTFA name: "phthalic anhydride/glycerol/glycidyl decanoate copolymer" and "adipic acid/neopentyl glycol/trimellitic anhydride copolymer", alkyds, tosylamide/formaldehyde condensates, polyurethanes and polyurea-urethanes, acrylic resins, silicone resins (non volatile and partially volatile).

In addition, the film-forming agent can be chosen from aqueous dispersions of polymer particles or film-forming latexes.

The aqueous dispersion comprising one film-forming polymer can be prepared by a person ordinary skill in the art on the basis of his or her general knowledge, for example, by emulsion polymerization or by dispersing the polymer formed beforehand.

Among the film-forming polymers which can be used in the composition, as disclosed herein, non-limiting mention may be made of: synthetic polymers, polycondensate type and radical type polymers, polymers of natural origin, and their blends.

Use may, for example, be made, but in the latex form, of the polymers (homo- and copolymers) which are mentioned above as polymers which are soluble or dispersible in an organic solvent medium and, for example, of the polymers of categories a, b and c.

Among polycondensates, non-limiting mention may, for example, be made of anionic, cationic, non-ionic or amphoteric polyurethanes, polyurethane-acrylics, polyurethane-polyvinylpyrrolidones, polyester-polyurethanes, polyether-polyurethanes, polyureas, polyurea-polyurethanes and of their blends.

Mention may also be made of polyesters, polyesteramides, polyesters with a fatty chain, polyamides and epoxy ester resins. For example, polyesters described above may be used.

Radical type polymers can for example, be chosen from acrylic and vinyl polymers and copolymers. For example, anionic radical polymers may be used. Mention may be made of monomers carrying an anionic group which can be used during the radical polymerization, of acrylic acid, methacrylic acid, crotonic acid, maleic anhydride and 2-acrylamido-2-methylpropanesulphonic acid.

The acrylic polymers can result from the copolymerization of monomers chosen from esters and amides of acrylic acid or of methacrylic acid. Non-limiting mention of monomers of the ester type include, for example, methyl methacrylate, ethyl methacrylate, butyl methacrylate, isobutyl methacrylate, 2-ethylhexyl methacrylate and lauryl methacrylate. As examples of monomers of amide type, mention may be made of N-(t-butyl)acrylamide and N-(octyl)acrylamide.

The vinyl polymers can result from the homopolymerization or from the copolymerization of monomers chosen from vinyl esters, styrene and butadiene. As examples of vinyl esters, non-limiting mention may be made of vinyl acetate, vinyl neodecanoate, vinyl pivalate, vinyl benzoate and vinyl t-butylbenzoate.

Use may also be made of acrylic/silicone copolymers and nitrocellulose/acrylic copolymers.

Mention may also be made of the polymers resulting from the radical polymerization of at least one radical monomer inside and/or partially on the surface of pre-existing particles of at least one polymer chosen from the group comprising of polyurethanes, polyureas, polyesters, polyesteramides and alkyds. These polymers are generally referred to as hybrid polymers.

The composition can also comprise an associative polymer of polyurethane type and natural gums, such as xanthan gum. -

Among polymers in aqueous dispersion, Non-limiting mention may be made of the dispersions of acrylic polymers sold under the names Neocryl XK-90^{®}, Neocryl A-1070^{®}, Neocryl A-1090^{®}, Neocryl BT-62^{®}, Neocryl A-1079^{®} and Neocryl A-523^{®} by Zeneca or Dow Latex 432^{®} by Dow Chemical. Use may also be made of aqueous polyurethane dispersions and, for example, the polyester-polyurethanes sold under the names "Avalure UR-405^{®}", "Avalure UR-410^{®}", "Avalure UR-425^{®}" or "Sancure 2060^{®}" by Goodrich and the polyether-polyurethanes sold under the names "Sancure 878^{®}" by Goodrich and "Neorez R-970^{®}" by Avecia.

In addition to those discussed above, the film-former can also be chosen from water-soluble and water-dispersible polymers.

Mention may be made, as water-dispersible polymers, of water-dispersible polycondensates with sulphonate functional groups, such as copolyesters composed of units derived from isophthalic acid, sodium salt of sulphoisophthalic acid, diethylene glycol, and 1,4-cyclohexanedimethanol. These polycondensates are sold under the names of "AQ 38/" and "AQ 55/" respectively by Eastman Kodak.

Among water-soluble polymers, non-limiting mention may be made of water-soluble copolymers with carboxylic acid functional groups (such as synthetic polymers) which are preferably chosen from: (a) polyoxyethylenated crotonic acid/vinyl acetate copolymers, (b) N-octylacrylamide/methyl methacrylate/hydroxypropyl methacrylate/acrylic acid/tert-butylaminoethyl methacrylate copolymers, (c) alternating methyl vinyl ether/maleic anhydride copolymers monoesterified by butanol, (d) acrylic acid/ethyl acrylate/N-(tert-butyl)acrylamide terpolymers, and (e) vinyl acetate/crotonic acid copolymers, vinyl acetate/crotonic acid/vinyl neodecanoate terpolymers, vinyl 4-(tert-butyl)benzoate and their blends.

The choice of the types of film-forming polymers used should depend on the type of physiologically acceptable medium chosen for the composition.

In one embodiment, the content of film-forming polymers can be present in an amount ranging from 0.1% to 15% by weight, for example, from 5% to 10% by weight, and further, for example, less than or equal to 7% by weight, relative to the total weight of the composition.

Other film forming agents can be chosen from any compound known to a person of ordinary skill in the art as being capable of fulfilling the desired function and can be chosen, for example, from plasticizers and coalescents for the film-forming polymer.

Mention may, for example, be made, alone or as a mixture, of conventional plasticizers and coalescents, such as: glycols and their derivatives, such as diethylene glycol ethyl ether, diethylene glycol methyl ether, diethylene glycol butyl ether, diethylene glycol hexyl ether, ethylene glycol ethyl ether, ethylene glycol butyl ether and ethylene glycol hexyl ether, glycol esters, propylene glycol derivatives and, for example, propylene glycol phenyl ether, propylene glycol diacetate, dipropylene glycol butyl ether, tripropylene glycol butyl ether, propylene glycol methyl ether, dipropylene glycol ethyl ether, tripropylene glycol methyl ether, diethylene glycol methyl ether and propylene glycol butyl ether, esters of acids, for example, carboxylic acids, such as citrates, in particular triethyl citrate, tributyl citrate, triethyl acetylcitrate, tributyl acetylcitrate or tri(2-ethylhexyl) acetylcitrate; phthalates, such as diethyl phthalate, dibutyl phthalate, dioctyl phthalate, dipentyl phthalate or dimethoxyethyl phthalate; phosphates, such as tricresyl phosphate, tributyl phosphate, triphenyl phosphate and tributoxyethyl phosphate; tartrates, such as dibutyl tartrate; adipates; carbonates; sebacates; benzyl benzoate; butyl acetylricinoleate; glyceryl acetylricinoleate; butyl glycolate; camphor; glyceryl triacetate; and N-ethyl-o,p-toluenesulphonamide, oxyethylenated derivatives, such as oxyethylenated oils, for example, vegetable oils, such as castor oil; and silicone oils, and their mixtures.

In one embodiment, the type and the amount of plasticizer and coalescent can be chosen by a person of ordinary skill in the art on the basis of his, or her, general knowledge, so as to obtain a composition cosmetically acceptable properties, with the proviso, that the total amount of texturizing agent does not exceed that indicated above and that the said composition retains its characteristic of fluidity and/or of ability to form a film having a low wear resistance

The composition may contain a thickening agent which can also serve as a stabilizer. The thickening agent can be chosen from: hydrophobic silicas, such as those disclosed in document EP-A-898 960 and, for example, sold under the references "Aerosil R812^{®}" by Degussa; "Cab-O-Sil TS-530^{®}", "Cab-O-Sil TS-610^{®}" and "Cab-O-Sil TS-720^{®}" by Cabot and "Aerosil R972^{®}" and "Aerosil R974^{®}" by Degussa; clays, such as montmorillonite, and modified clays, such as bentonites, for example stearalkonium hectorite and stearalkonium bentonite, and polysaccharide alkyl ethers (for example, wherein the alkyl group comprises from 1 to 24 carbon atoms, such as from 1 to 10, further, for example, from 1 to 6 carbon atoms, and, for example, from 1 to 3 carbon atoms), such as those disclosed in the document EP-A-898 958.

The thickening agent can also be chosen from thickeners for an aqueous phase, such as aqueous gelling polymers, gums such as xantham gum, hydrated silica and clays such as bentonite and hectorite.

The total proportion of thickening agents in the compositions, as disclosed herein, is generally less than or equal to 5% by weight, for example less than or equal to 2% by weight, and further, for example, less than or equal to 1% by weight, relative to the total weight of the composition.

The composition may also optionally include a carrier (f) which serves to place the composition into a physical form usable for the intended use. The carrier can be oils or waxes as described. The carrier can be a solvent such as water, a water/solvent mixture of an alcohol or other organic solvent. Non-limiting examples of solvents that are miscible with water include ethanol, isopropanol, propanol, butanol, isobuthanol, glycols comprising from 2-8 carbon atoms, such as propylene glycol, ethylene glycol, 1,3-butylene glycol, dipropylene glycol, ketones comprising 3-4 carbon atoms such a acetone and methylethylketone, and aldehydes containing 2-4 carbon atoms.

Alcohol and other organic solvents include ketones which are liquid at ambient temperature, such as methyl ethyl ketone, methyl isobutyl ketone, diisobutyl ketone, isophorone, cyclohexanone and acetone; alcohols which are liquid at ambient temperature, such as ethanol, isopropanol, butanol, diacetone alcohol, 2-butoxyethanol and cyclohexanol; glycols which are liquid at ambient temperature, such as ethylene glycol, propylene glycol, pentylene glycol and glycerol; propylene glycol ethers which are liquid at ambient -temperature, such as propylene glycol monomethyl ether, propylene glycol monomethyl ether acetate and dipropylene glycol mono(n-butyl) ether; short-chain esters (comprising from 3 to 8 carbon atoms), such as methyl acetate, ethyl acetate, propyl acetate, n-butyl acetate, and isopentyl acetate and aryl acetate; alkanes which are liquid at ambient temperature, such as decane, heptane, octane, dodecane, cyclohexane and isododecane; aldehydes which are liquid at ambient temperature, such as benzaldehyde and acetaldehyde, and their mixtures.

Typical film formers include polyvinylpyyrolidone, polysilicone 8, polydimethylsiloxane, dimethylsiloxane/3-thiopropyl methyl siloxane copolymer, vinylpyyrolidone/vinylacetate copolymer, polyvinyacetate, starch, cellulose, polyquaternium-4, polyquaternium-11, acrylates/steareth-2 methacrylate crosspolymer, vinylacetate/vinyl neodecanoate copolymer, polyester-5, cetyl ethylhexanoate, vinyl acetate, crotonate/vinyl neodecanoate copolymer, 2-acryamido-2-methyl propane sulfonic acid (AMPS)/acrylic acid (AA) copolymer, AMPS/AA/acryl methacrylate copolymer, polyacrylamide, C₁₃-C₁₄ isoparaffin, laureth-7, octylacrylamide, acrylate/butylaminoethylmethacrylate copolymer and mixtures thereof.

Typical stabilizers include acrylate/steareth-20 methacrylate crosspolymer, carbomer, acrylic acid, polyacrylic acid crosslinked with allyl ether pentaerythritol, polyacrylic acid crosslinked with an allyl ether of sucrose, polyacrylic acid crosslinked with an allyl ether of propylene, isoceteth-20, hydroxyethylcellulose, acrylate/beheneth-25/methacrylate copolymer, PEG-40 hydrogenated castor oil, steareth-20, PPG-5-ceteth-20, oleth-10 phosphate, polyacrylamide, C₁₃-C₁₄ isoparaffin, laureth-7, isoceteth-20, cocamidopropyl betaine, glyceryl laurate, disodium cocoamphodiproprionate, PEG-150 distearate, 6000 distearate, PEG-200 glyceryl stearate, PEG-40 hydrogenated castor oil, polyacrylate-3, hydroxyethylcellulose, PEG-10, hydroxypropyl guar, laureth-4, cetrimonium chloride, polyoxyl 40 hydrogenated castor oil, polyquaternium-37, propylene glycol dicaprylate/dicaprate, PPG-1 , trideceth-6, Peg-14 dimethicone, bisamino PEG/PPG-41/3 aminoethyl PG-propyl dimethicone, polysorbate 20, oleth-20, stearyl alcohol, cetyl alcohol, dimethicone and mixtures thereof.

Typical auxiliaries include caprylyl glycol, glycerin, propylene glycol, isobutene, propane, hexylene glycol, alklene glycol, dimethicone and mixtures thereof.

The disclosure also relates to a method for treating hair which first involves formulating a suitable composition with the composition of the disclosure. The suitable composition is then applied to the hair. The specific suitable composition contains components of the disclosed composition in a concentration which varies for each of these applications. Hence, the compositions of the invention can be used for treating hair. Methods for altering hair colour or for dyeing hair comprising applying the compositions of the invention to the hair are encompassed by the invention. Further, the compositions can be used to cosmetically treat hair e.g. to improve feel, manageability, protect from damage etc.

The disclosure also relates to a method for treating hair, skin, lips, eyes and nails which first involves formulating a suitable composition with the composition of the disclosure. The suitable composition is then applied to the hair, skin, lips, eyes and nails. The specific suitable composition contains components of the disclosed composition in a concentration which varies for each of these applications. Hence, the compositions of the invention are for use in treating hair, skin, lips, eyes and nails. The compositions can be used to alter skin colour e.g. to tan the skin and methods of tanning are encompassed by the invention. The compositions can further be used to alter the colour of lips or to improve their appearance in any other way e.g. by using cosmetics such as lipsticks, gloss, lip liner etc comprising the composition of the invention and applying the cosmetic to the lips. The eyes e.g. eye lids, eye lashes, eye brows and under eye area can be treated with the compositions of the invention to alter their colour or appearance. For example, the composition of the invention can be used as or in cosmetic products such as eye shadow, eye liner, mascara or can be used to alter the colour of the eye brows or eye lashes e.g. by dyeing. With regard to nails, the compositions can be used in the form of nail varnish to alter to the colour of nails and/or to improve their condition.

The following examples are for illustrative purposes only and are not intended to limit the scope of the claims. The examples are prepared by mixing the components using techniques standard in the cosmetics industry.

A cosmetic gel and a cosmetic mousse composition were prepared in accordance with the disclosure. The components of the compositions are listed in Formula I and Formula II in the following tables. The functionalized metal-oxide pigment for each was a silica/iron oxide layered pigment functionalized with a PEG-DOPA polymer substituted catachol compound.

### FORMULA 1 (MOUSSE)

| **INGREDIENT (INCI NAME)** | **% BY WEIGHT OF THE COMPOSITION** |
|---|---|
| PROPYLENE GLYCOL | 2.35 |
| *PIGMENT (INDIAN SUMMER;SILICA AND IRON OXIDES) | 1.88 |
| PEG-40 | 0.18 |
| POLYQUATERNIUM-37 | 0.71 |
| LAURETH-4 | 0.38 |
| GLYCERIN | 4.70 |
| PPG-1 TRIDECETH-6 | 0.10 |
| PEG-40 HYDROGENATED CASTOR OIL | 0.56 |
| PROPYLENE GLYCOL DICAPRYLATE | 0.49 |
| POLYQUATERNIUM-4 | 1.88 |
| PROPELLANT (ISOBUTANE AND PROPANE) | 6.00 |
| CETRIMONIUM CHLORIDE | 0.05 |
| PRESERVATIVES | 0.66 |
| WATER | q.s. to 100% |

| | |
|---|---|
| *Surface functionalized pigment (Indian summer; silica and iron oxides) with dopamine-based polymer (Seng et al., J. Am. Chem. Soc., 2006, 126, 10676-10677): | |

### FORMULA 2 (GEL)

| **INGREDIENT (INCI NAME)** | **% BY WEIGHT OF THE COMPOSITION** |
|---|---|
| POLYSILICONE-8 | 0.16 |
| POLYACRYLATE-3 | 0.63 |
| TRIETHANOLAMINE | 0.72 |
| *PIGMENT (INDIAN SUMMER;SILICA AND IRON OXIDES) | 2.00 |
| PVP | 3.00 |
| PEG-40 | 0.12 |
| PEG-40 HYDROGENATED CASTOR OIL | 0.38 |
| PROPYLENE GLYCOL | 2.50 |
| ALCOHOL DENAT. | 1.80 |
| HYDROXYETHYLCELLULOSE | 0.02 |
| PRESERVATIVES | 0.90 |
| WATER | q.s. to 100% |

| | |
|---|---|
| **Surface functionalized pigment (Indian summer; silica and iron oxides) with dopamine-based polymer (Seng et al., J. Am. Chem. Soc., 2006, 126, 10676-10677). | |

### FORMULA A (MOUSSE)

| **INGREDIENT (INCI NAME)** | **% BY WEIGHT OF THE COMPOSITION** |
|---|---|
| BISAMINO PEG/PPG-41/3 AMINOETHYL PG-PROPYL DIMETHICONE | 0.20 |
| DIPROPYLENE GLYCOL | 0.46 |
| STEARYL ALCOHOL | 0.14 |
| PIGMENT (XIRONA^{®} INDIAN SUMMER;SILICA AND IRON OXIDES) | 1.88 |
| POLYQUATERNIUM-37 | 0.71 |
| CETYL ALCOHOL | 0.047 |
| POLYQUATERNIUM-11 | 1.504 |
| GLYCERIN | 0.376 |
| PROPYLENE GLYCOL | 0.65 |
| PPG-1 TRIDECETH-6 | 0.10 |
| COCAMIDOPROPYL BETAINE | 0.66 |
| PEG-14 DIMETHICONE | 0.56 |
| PROPYLENE GLYCOL DICAPRYLATE/DICAPRATE | 0.49 |
| OLETH-20 | 0.85 |
| POLYSORBATE 20 | 0.56 |
| PROPELLANT (ISOBUTANE AND PROPANE) | 6.00 |
| COSMETIC ADDITIVES SUCH AS VEGETABLE EXTRACTS, VITAMINS, FRAGRANCE, CITRIC ACID | 0.77 |
| PRESERVATIVES | 1.04 |
| WATER | q.s. to 100% |

### FORMULA B (MOUSSE)

| **INGREDIENT (INCI NAME)** | **% BY WEIGHT OF THE COMPOSITION** |
|---|---|
| BISAMINO PEG/PPG-41/3 AMINOETHYL PG-PROPYL DIMETHICONE | 0.20 |
| DIPROPYLENE GLYCOL | 0.46 |
| STEARYL ALCOHOL | 0.14 |
| PIGMENT (XIRONA^{®}NORDIC SUNSET; SILICA AND TITANIUM DIOXIDE) | 1.88 |
| POLYQUATERNIUM-37 | 0.71 |
| CETYL ALCOHOL | 0.047 |
| POLYQUATERNIUM-11 | 1.504 |
| GLYCERIN | 0.376 |
| PROPYLENE GLYCOL | 0.65 |
| PPG-1 TRIDECETH-6 | 0.10 |
| COCAMIDOPROPYL BETAINE | 0.66 |
| PEG-14 DIMETHICONE | 0.56 |
| PROPYLENE GLYCOL DICAPRYLATE/DICAPRATE | 0.49 |
| OLETH-20 | 0.85 |
| POLYSORBATE 20 | 0.56 |
| PROPELLANT (ISOBUTANE AND PROPANE) | 6.00 |
| COSMETIC ADDITIVES SUCH AS VEGETABLE EXTRACTS, VITAMINS, FRAGRANCE, CITRIC ACID | 0.77 |
| PRESERVATIVES | 1.04 |
| WATER | q.s. to 100% |

### FORMULA C (MOUSSE)

| **INGREDIENT (INCI NAME)** | **% BY WEIGHT OF THE COMPOSITION** |
|---|---|
| PROPYLENE GLYCOL | 2.35 |
| PIGMENT (XIRONA^{®} INDIAN SUMMER;SILICA AND IRON OXIDES) | 1.88 |
| PEG-40 | 0.18 |
| POLYQUATERNIUM-37 | 0.71 |
| LAURETH-4 | 0.38 |
| GLYCERIN | 4.70 |
| PPG-1 TRIDECETH-6 | 0.10 |
| PEG-40 HYDROGENATED CASTOR OIL | 0.56 |
| PROPYLENE GLYCOL DICAPRYLATE/DICAPRATE | 0.49 |
| POLYQUATERNIUM-4 | 1.88 |
| PROPELLANT (ISOBUTANE AND PROPANE) | 6.00 |
| CETRIMONIUM CHLORIDE | 0.05 |
| PRESERVATIVES | 0.66 |
| WATER | q.s. to 100% |

### FORMULA D (MOUSSE)

| **INGREDIENT (INCI NAME)** | **% BY WEIGHT OF THE COMPOSITION** |
|---|---|
| PROPYLENE GLYCOL | 2.35 |
| PIGMENT (XIRONA^{®} NORDIC SUNSET; SILICA AND TITANIUM DIOXIDE) | 1.88 |
| PEG-40 | 0.18 |
| POLYQUATERNIUM-37 | 0.71 |
| LAURETH-4 | 0.38 |
| GLYCERIN | 4.70 |
| PPG-1 TRIDECETH-6 | 0.10 |
| PEG-40 HYDROGENATED CASTOR OIL | 0.56 |
| PROPYLENE GLYCOL DICAPRYLATE/DICAPRATE | 0.49 |
| POLYQUATERNIUM-4 | 1.88 |
| PROPELLANT (ISOBUTANE AND PROPANE) | 6.00 |
| CETRIMONIUM CHLORIDE | 0.05 |
| PRESERVATIVES | 0.66 |
| WATER | q.s. to 100% |

### FORMULA E (GEL)

| **INGREDIENT (INCI NAME)** | **% BY WEIGHT OF THE COMPOSITION** |
|---|---|
| POLYSILICONE-8 | 0.16 |
| POLYACRYLATE-3 | 0.63 |
| TRIETHANOLAMINE | 0.72 |
| PIGMENT (XIRONA^{®} INDIAN SUMMER;SILICA AND IRON OXIDES) | 2.00 |
| PVP | 3.00 |
| PEG-40 | 0.12 |
| PEG-40 HYDROGENATED CASTOR OIL | 0.38 |
| PROPYLENE GLYCOL | 2.50 |
| ALCOHOL DENAT. | 1.80 |
| HYDROXYETHYLCELLULOSE | 0.02 |
| PRESERVATIVES | 0.90 |
| WATER | q.s. to 100% |

### FORMULA F (GEL)

| **INGREDIENT (INCI NAME)** | **% BY WEIGHT OF THE COMPOSITION** |
|---|---|
| POLYSILICONE-8 | 0.16 |
| POLYACRYLATE-3 | 0.63 |
| TRIETHANOLAMINE | 0.72 |
| PIGMENT (XIRONA^{®}NORDIC SUNSET; SILICA AND TITANIUM DIOXIDE) | 2.00 |
| PVP | 3.00 |
| PEG-40 | 0.12 |
| PEG-40 HYDROGENATED CASTOR OIL | 0.38 |
| PROPYLENE GLYCOL | 2.50 |
| ALCOHOL DENAT. | 1.80 |
| HYDROXYETHYLCELLULOSE | 0.02 |
| PRESERVATIVES | 0.90 |
| WATER | q.s. to 100% |

### FORMULA G (HAIR LOTION)

| **INGREDIENT (INCI NAME)** | **% BY WEIGHT OF THE COMPOSITION** |
|---|---|
| POLYACRYLATE-3 | 0.25 |
| TRIETHANOLAMINE | 0.25 |
| PIGMENT (XIRONA^{®} INDIAN SUMMER;SILICA AND IRON OXIDES) | 2.00 |
| VP/VA COPOLYMER | 2.50 |
| PEG-40 | 0.10 |
| HYDROXYPROPYL GUAR | 0.25 |
| PROPYLENE GLYCOL | 1.00 |
| PEG-40 HYDROGENATED CASTOR OIL | 0.30 |
| PRESERVATIVES | 0.90 |
| WATER | q.s. to 100% |

### FORMULA H (HAIR LOTION)

| **INGREDIENT (INCI NAME)** | **% BY WEIGHT OF THE COMPOSITION** |
|---|---|
| POLYACRYLATE-3 | 0.25 |
| TRIETHANOLAMINE | 0.25 |
| PIGMENT (XIRONA^{®}NORDIC SUNSET; SILICA AND TITANIUM DIOXIDE) | 2.00 |
| VP/VA COPOLYMER | 2.50 |
| PEG-40 | 0.10 |
| HYDROXYPROPYL GUAR | 0.25 |
| PROPYLENE GLYCOL | 1.00 |
| PEG-40 HYDROGENATED CASTOR OIL | 0.30 |
| PRESERVATIVES | 0.90 |
| WATER | q.s. to 100% |

### FORMULA I (STYLING GEL)

| **Ingredient** | Percentage |
|---|---|
| Xirona® Nordic Sunset Silica and Titanium Dioxide | 1.50% |
| Acrylates/Steareth-20 Methacrylate Crosspolymer | 1.00% |
| Vinylpyrrolidone/Dimethylaminoethylmethacrylate Copolymer | 0.50% |
| Propylene Glycol | 5.00% |
| Water | 92.00% |

### FORMULA J (STYLING GEL)

| **Ingredient** | **Percentage** |
|---|---|
| Xirona® Nordic Sunset Silica and Titanium Dioxide | 1.50% |
| Acrylates/Steareth-20 Methacrylate Crosspolymer | 2.00% |
| Propylene Glycol | 7.00% |
| Water | 89.50% |

### FORMULA K (STYLING GEL)

| **Ingredient** Percentage | |
|---|---|
| Xirona® Nordic Sunset Silica and Titanium Dioxide | 1.50% |
| Acrylates/Steareth-20 Methacrylate Crosspolymer | 1.00% |
| Ethylenediamine | 1.00% |
| Propylene Glycol | 5.00% |
| Water | 91.50% |

### FORMULA L (STYLING GEL)

| **Ingredient** | Percentage |
|---|---|
| Xirona® Indian summer Silica and Iron Oxides | 1.50% |
| Acrylates/Steareth-20 Methacrylate Crosspolymer | 2.00% |
| Propylene Glycol | 7.00% |
| Water | 89.50% |

### FORMULA M (STYLING GEL)

| **Ingredient** | Percentage |
|---|---|
| Xirona® Le Rouge Iron Oxides and Silica | 1.50% |
| Acrylates/Steareth-20 Methacrylate Crosspolymer | 2.00% |
| Propylene Glycol | 7.00% |
| Water | 89.50% |

### FORMULA N (STYLING MOUSSE)

| **Ingredient** | percentage |
|---|---|
| Xirona® Nordic Sunset Silica and Titanium Dioxide | 1.88% |
| AMP-Acrylares/Allyl Methacrylate Copolymer | 0.94% |
| Vinylpyrrolidone/Dimethylaminoethylmethacrylate Copolymer | 0.94% |
| Sulfate (and) Magnesium Laureth Sulfate (and) Sodium Oleth Sulfate (and) Magnesium Oleth Sulfate | 0.19% |
| Isobutane/Propane | 6.00% |
| Water | 90.05 |

### FORMULA O (STYLING GEL)

| **INGREDIENT (INCI NAME)** | **% BY WEIGHT OF THE COMPOSITION** |
|---|---|
| Xirona® Indian Summer | 2.00 |
| Phenoxyethanol | 0.50 |
| Polyurethane-6 | 0.3 |
| Aminomethylpropanol | 1.00 |
| PEG-40 Hydrogenated castor oil | 0.50 |
| Laureth-7 | 0.077 |
| PEG-200 Glyceryl stearate | 1.60 |
| Alcohol denat. | 0.10 |
| VA/Vinyl Butyl Benzoate/Crotonates Copolymer | 10.00 |
| C13-14 Isoparaffin | 0.231 |
| Fragrance | 0.30 |
| PRESERVATIVES | 0.20 |
| Polyacrylamide | 0.44 |
| PEG -150 Distearate | 2.40 |
| WATER | q.s. to 100% |

Next, several experiments are conducted to demonstrate the efficacy of the disclosed composition. The results of the experiments are summarized in Table 1. In the experiments, several formulations are prepared with various amounts of film-former, stabilizer and auxiliary components. The formulations are tested on hair for pigment transfer properties, pigment dust effect. The samples are rated on a five step rating from low to high (low, low-medium, medium, medium-high and high). The pigment transfer property is a subjective measure of how easily the pigment is transferred from the hair after the composition is applied to the hair. The transfer property is measured by handling the hair and evaluating how much pigment transfers from the hair. A low pigment transfer effect is desirable whereas a high transfer effect is undesirable.

The color travel effect is a subjective measure of how much the color of the treated hair shifts depending upon viewing angle (color travel effect). The same five step rating described above is utilized; however, a low color travel effect is undesirable and a high effect is most desirable.

The pigment dust effect is a subjective measure of how much pigment dust is generated when the hair is handled after the disclose composition is applied to the hair. The five step rating described above is utilized. A low pigment dust effect is most desirable and high dust effect is most undesirable. The results of a study demonstrating the efficacy of the disclosed composition in cosmetic hair formulations is summarized in Table 1.

**Table 1**

| Type of Hair Formulation | | Composition¹ (wt%) | | | Pigment² Transfer Properties | Pigment³ Color Travel Effect | Pigment⁴ Dust Effect |
|---|---|---|---|---|---|---|---|
| | Pigment | Film Former | Stabilizer | Auxiliary | | | |
| | | | | | | | |
| Gel | Xirona^{®} Indian Summer | 3.2% | 0.6% | 2.5% | Low Medium | Medium | Low |
| Gel | Xirona^{®} Nortic Sunset | 3.2% | 0.6% | 2.5% | Low Medium | Medium-high | Low |
| Mousse | Xirona^{®} Nortic Sunset | 1.9% | 1.3% | 7.0% | Low | Medium | Low |
| Mousse | Xirona^{®} Nortic Sunset | 1.5% | 4.2% | 0.9% | Low | Medium | Low |
| Gel | Xirona^{®} Indian Summer | 0% | 2.5% | 48% | High | Low | Low-medium |
| Gel | Xirona^{®} Indian Summer | 5.0% | 0.5% | 0% | Low-Medium | Low-Medium | Medium-high |
| Mousse | Xirona^{®} Indian Summer | 1.0% | 0% | 2.4% | High | Medium | High |

| | | | | | | | |
|---|---|---|---|---|---|---|---|
| 1. The balance of ingredients include water and other components necessary to complete the type of hair formulation. 2. Pigment transfer is a measure of transfer of pigment from the hair (Low=most desirable, High=least desirable) 3. Pigment travel effect is the optical effect of color shift of the pigment on the hair (Low=least desirable, High=most desirable) 4. Pigment dust effect is the amount of dust generated from the pigment from the hair in everyday use (Low=most desirable, High=least desirable). | | | | | | | |

Inspection of Table 1, shows that in the absence of one of the auxiliary, stabilizer or film former components, the cosmetic hair formulation has poor pigment transfer and poor pigment dust properties. These results demonstrate the unexpected efficacy of the disclosed composition in cosmetic hair compositions.

The foregoing description illustrates and describes the present disclosure. Additionally, the disclosure shows and describes only the preferred embodiments of the disclosure, but, as mentioned above, it is to be understood that it is capable of changes or modifications within the scope of the concept as expressed herein, commensurate with the above teachings and/or skill or knowledge of the relevant art. The embodiments described hereinabove are further intended to explain best modes known of practicing the disclosure and to enable others skilled in the art to utilize the disclosure in such, or other, embodiments and with the various modification required by the particular applications or uses disclosed herein. Accordingly, the description is not intended to limit the disclosure to the form disclosed herein. Also, it is intended that the appended claims be construed to include alternative embodiments.

All publications, patents and patent applications cited in this specification are herein incorporated by reference, and for any and all purposes, as if each individual publication, patent or patent application were specifically and individually indicated to be incorporated by reference. In the case of inconsistencies, the present disclosure will prevail.

## Claims

1. A composition comprising:
(a) a metal-oxide layered pigment;
(b) optionally, at least one film-former;
(c) at least one stabilizer and
(d) at least one auxiliary ingredient
wherein the metal-oxide layered pigment comprises at least two layers and wherein the optional at least film one film-former when present and the at least one stabilizer are each added at a concentration of about 0.05 to about 15 weight % based on the weight of the composition.

2. The composition as claimed in claim 1 further comprising:
(e) at least one polymer and
wherein the surface of the metal-oxide layered pigment is functionalized with a ligand by formation of at least one bond between a metal at the surface of the metal-oxide layered pigment and the ligand.

3. The composition as claimed in claims 1 or 2, wherein the metal-oxide layered pigment comprises at least one metal oxide selected from the group consisting of TiO₂, Fe₂O₃, CaCO₃, SnO, SnO₂ MgSiO₃, Cr₂O₃, ZnO, MgO, ZnS, ZrO₂, CuO, MgF₂, Ce₂O₃, CeO₂, Y₂O₃, CaF₂, Al₂O₃, BaSO₄, BiOCI, SiO₂, and glass flake mica, talc and kaolin and optionally comprising a polymer particle.

4. The composition as claimed in any one of claims 1 to 3, wherein, the at least one film-former when present and at least one stabilizer are selected from the group consisting of an anionic compound, a non-ionic compound, an amphoteric compound, a zwitterionic compound, a protein, a viscosity modifier, a cationic polymer, a polyacrylate, a polymethacrylate, a polyacrylate copolymer, a polymethacrylate copolymer, a polyamine, polyaminoamide, polyester, a silicone resin, a polysaccharide, a starch, a gum, a silicone fluid, a polyacryamide, a polyacrylamide/polyacrylate copolymer and mixtures thereof.

5. The composition as claimed in any one of claims 1 to 4, wherein the at least one auxiliary ingredient is selected from the group consisting of an anionic surfactant, a non-ionic surfactant, an amphoteric surfactant, a zwitterionic surfactant, a fatty carboxylic acid, and its salt a fatty ether carboxylic acid, and its salt a fatty phosphoric acid and its salt, a fatty ether carboxylic acid and its salt, a polyamine, an oil, a fatty ester, a hydrocarbon, a silicone, a wax, a fatty alcohol, an antibacterial agent, a sunscreen, a preservative, a pH adjusting agent, a bleaching agent, a perfume, a sequestering agent, a fragrance, a coloring agent, a film forming agent, an amino acid, a cationic conditioner, a water insoluble ingredient, a filler, a coloring agent and mixtures thereof.

6. The composition as claimed in any one of claims 1 to 5, further comprising a carrier (f) wherein the carrier is at least one selected from the group consisting of water, an alcohol, a water/alcohol mixture, a water/organic solvent mixture and an organic solvent.

7. The composition as claimed in any one of claims 1 to 6, wherein the metal-oxide layered pigment comprises from 3 to 7 layers.

8. The composition as claimed in any one of claims 1 to 7, wherein the metal-oxide layered pigment is selected from the group of pigments marketed under the trade designations XIRONA^{®}, COLORONA^{®}, TIMIRON^{®}, DICHRONA^{®}, MICRONA^{®}, SOLORON^{®}, PRESTIGE^{®}, FLONAC^{®}, FLAMENCO^{®}, TIMICA^{®}, DUOCHROME^{®} and mixtures thereof.

9. The composition as claimed in claim 2, wherein the ligand is at least one compound selected from the group consisting of catechol, a substituted catechol, electron-donating bidentate ligands, dihydroxy cyclobutenedione, ascorbic acid, methyl catechol, tertbutyl catechol, salicylic acid, a substituted salicylic acid, gallol, a substituted gallol, benzene-1,2,3-triol, a substituted benzene-1,2,3-triol, benzene-1,2,4-triol, a substituted benzene-1,2,4-triol, benzene-1,2,3,4-tetraol, a substituted benzene-1,2,3,4-tetraol, dopamine, alizarin, an organoamine, an organopolycarboxylate, an organoamino carboxylate, an organosilane, an alkoxysilane, an organophosphate, an organophosphonate, an aminophosphonate, a disulfide, a mercaptocarboxylate, an alkylthiocarbamate, an alkylcarbamate, a polyalkylene glycol/amino acid copolymer, a mPEG-DOPA copolymer, poly[(3,4-dihydroxystyrene)/styrene copolymer, a polyalkylene glycol functionalized acrylic acid, a polyalkylene glycol functionalized methacrylic acid, a gallol-PEG polymer, a compound or polymer with adjacent hydroxyl groups and mixtures thereof, or the ligand is at least one compound selected from the group consisting of dihydroxy cyclobutenedione, ascorbic acid, catechol, methyl catechol, t-butyl catechol, dopamine, alizarin, polyethylene glycol/dihydroxyphenylalanine copolymer, a mPEG-DOPA copolymer, poly[(3,4-dihydroxystyrene)/styrene copolymer, aminopropyltriethyoxysilane, ammoniumpropyltriethoxysilane, trimethylammoniumpropyltriethoxylilane and mixtures thereof.

10. The composition as claimed in claim 2, wherein the at least one polymer is selected from the group consisting of an anionic surfactant, a nonionic surfactant, an amphoteric surfactant, a zwitterionic surfactant, a protein, a viscosity modifier, a cationic polymer, a polyacrylate, a polymethacrylate, a polyacrylate copolymer, a polymethacrylate copolymer, a polyamine, polyaminoamide, polyester, a silicone resin, a polysaccharide, a starch, a gum, a silicone fluid, a polyacryamide, a polyacrylamide/polyacrylate copolymer and mixtures thereof.

11. The composition as claimed in any one of claims 1 to 10, wherein the individual layers of the metal-oxide layered pigment have a thickness of from about 0.02 to about 2 microns.

12. The composition as claimed in any one of claims 1 to 11, wherein the particle size of the metal-oxide layered pigment is from about 5 to about 500 microns or from about 10 to about 100 microns.

13. A method for treating hair comprising applying the composition as claimed in any one of claims 1 to 12 to hair.

14. A method for treating skin, lips, eyes or nails comprising applying the composition as claimed in any one of claims 2 to 12 to skin, lips, eyes or nails.

15. The composition as claimed in any one of claims 1 to 12, wherein the at least one film former is present and is selected from the group consisting of polyvinylpyyrolidone, polysilicone 8, polydimethylsiloxane, dimethylsiloxane/3-thiopropyl methyl siloxane copolymer, vinylpyyrolidone/vinylacetate copolymer, polyvinyacetate, starch, cellulose, polyquaternium-4, polyquaternium-11, acrylates/steareth-2 methacrylate crosspolymer, vinylacetate/vinyl neodecanoate copolymer, polyester-5, cetyl ethylhexanoate, vinyl acetate, crotonate/vinyl neodecanoate copolymer, 2-acryamido-2-methyl propane sulfonic acid (AMPS)/acrylic acid (AA) copolymer, AMPS/AA/acryl methacrylate copolymer, polyacrylamide, C₁₃-C₁₄ isoparaffin, laureth-7, octylacrylamide, acrylate/butylaminoethylmethacrylate copolymer and mixtures thereof.

16. The composition as claimed in any one of claims 1 to 12 or 15, wherein the at least one stabilizer is selected from the group consisting of acrylate/steareth-20 methacrylate crosspolymer, carbomer, acrylic acid, polyacrylic acid crosslinked with allyl ether pentaerythritol, polyacrylic acid crosslinked with an allyl ether of sucrose, polyacrylic acid crosslinked with an allyl ether of propylene, isoceteth-20, hydroxyethylcellulose, acrylate/beheneth-25/methacrylate copolymer, PEG-40 hydrogenated castor oil, steareth-20, PPG-5-ceteth-20, oleth-10 phosphate, polyacrylamide, C₁₃-C₁₄ isoparaffin, laureth-7, isoceteth-20, cocamidopropyl betaine, glyceryl laurate, disodium cocoamphodiproprionate, PEG-150 distearate, 6000 distearate, PEG-200 glyceryl stearate, PEG-40 hydrogenated castor oil, polyacrylate-3, hydroxyethylcellulose, PEG-10, hydroxypropyl guar, laureth-4, cetrimonium chloride, polyoxyl 40 hydrogenated castor oil, polyquaternium-37, propylene glycol dicaprylate/dicaprate, PPG-1 trideceth-6, Peg-14 dimethicone, bisamino PEG/PPG-41/3 aminoethyl PG-propyl dimethicone, polysorbate 20, oleth-20, stearyl alcohol, cetyl alcohol, dimethicone and mixtures thereof.

17. The composition as claimed in any one of claims 1 to 12, 15 or 16, wherein the at least one auxiliary ingredient is selected from the group consisting of caprylyl glycol, glycerin, propylene glycol, isobutene, propane, hexylene glycol, alklene glycol, dimethicone and mixtures thereof.

18. The composition as claimed in claim 2, wherein the ligand is a substituted catechol and the catechol is substituted with a group selected from the group consisting of amino, alkyl amino, N-substituted amino, -O-alkyl, -O-aryl, alkyl, alkenyl, alkynyl, cycloalkyl, heterocycloalkyl, aryl, heteroaryl, alkylaryl, alkyl-substituted cycloalkyl, alkyl-substituted heterocycloalkyl, araalkyl, alkylheteroaryl, heteroarylalkyl, a mPEG-DOPA oligomer or polymer and a polyalkyl glycol oligomer or polymer or wherein the ligand is a substituted salicylic acid and the salicylic acid is substituted with a group selected from the group consisting of amino, alkyl amino, N-substituted amino, -O-alkyl, -O-aryl, alkyl, alkenyl, alkynyl, cycloalkyl, heterocycloalkyl, aryl, heteroaryl, alkylaryl, alkyl-substituted cycloalkyl, alkyl-substituted heterocycloalkyl,ara alkyl, alkylheteroaryl, heteroarylalkyl, a mPEG-DOPA oligomer or polymer and a polyalkyl glycol oligomer or polymer.
